# FASCICULE DE BREVET EUROPEEN

(11) **EP 2 856 154 B1**
(45) Date de publication et mention de la délivrance du brevet: **14.03.2018**
(21) Numéro de dépôt: 13731837.4
(22) Date de dépôt: 28.05.2013
(51) Int. Cl.: G01N 33/50, G01N 33/68

(54) **MÉTHODES DE DÉCONTAMINATION DES CIRCUITS DE PRODUCTION DE POLYMÈRES DE GLUCOSE ET D'HYDROLYSATS DE POLYMÈRES DE GLUCOSE**
VERFAHREN ZUR DEKONTAMINATION VON KREISLÄUFEN ZUR HERSTELLUNG VON GLUCOSEPOLYMEREN UND HYDROLYSATEN VON GLUCOSEPOLYMEREN
METHODS FOR DECONTAMINATING CIRCUITS FOR PRODUCING GLUCOSE POLYMERS AND HYDROLYSATES OF GLUCOSE POLYMERS

(30) Priorité: 29.05.2012 FR 1254935; 16.07.2012 FR 1256848; 18.10.2012 FR 1259923; 16.01.2013 FR 1350353; 27.03.2013 FR 1352748
(43) Date de publication de la demande: 08.04.2015
(73) Titulaire: Roquette Frères, 62136 Lestrem (FR)
(72) Inventeur: DUVET, Sophie, F-59890 Quesnoy Sur Deule (FR); HACINE-GHERBI, Héla, F-59650 Villeneuve D'ascq (FR); LANOS, Pierre, F-59480 La Bassee (FR); ALLAIN, Fabrice, F-59800 Lille (FR); CARPENTIER, Mathieu, F-59350 Saint Andre Lez Lille (FR); DENYS, Agnès, F-59800 Lille (FR)
(74) Mandataire: Gallois, Valérie
(86) Numéro de dépôt international: PCT/FR2013/051181
(87) Numéro de publication internationale: WO 2013/178931

(56) Documents cités:
- WO-A1-2007/076411
- WO-A1-2010/125315
- WO-A1-2012/143647
- FR-A1- 2 978 774
- US-A1- 2005 191 717
- US-A1- 2009 239 819

## Description

La présente invention est relative à des méthodes de décontamination des circuits de production ou de purification de polymères de glucose, plus particulièrement ceux destinés aux domaines alimentaires (ingrédients santés riches en fibres) et médicaux (dialyse péritonéale), ou d'hydrolysats de polymères de glucose, plus particulièrement ceux destinés aux domaines médicaux (glucose apyrogène injectable).

### Arrière-plan technologique de l'invention

La société Demanderesse a choisi de développer son invention dans un domaine connu pour la dangerosité des contaminants d'origine microbienne susceptibles de se développer dans les circuits de production des polymères de glucose ou dans ceux de production de leurs hydrolysats, contaminants à l'origine de possibles :
- intoxications alimentaires,
- réactions inflammatoires très néfastes pour la santé humaine.

Dans le cadre d'une démarche de sécurité alimentaire, comme dans celui d'une démarche de sécurité sanitaire, il est donc important de s'assurer de l'absence de contaminants d'origine microbienne, tant sous forme de cellules vivantes que de débris cellulaires, par tous les moyens techniques adéquats, notamment :
- la définition de circuits de production sécurisés, par la mise en place de dispositifs et de techniques de purification adaptées,
- la définition de méthodes d'identification et de dosage efficaces des contaminants.

Par exemple, dans le cas de la dialyse péritonéale, un certain nombre d'ingrédients doivent être préparés dans les conditions de pureté les plus strictes.

La dialyse péritonéale est en effet un type de dialyse qui a pour objectif d'éliminer les déchets tels que l'urée, la créatinine, l'excès de potassium ou l'excédent d'eau que les reins ne parviennent pas ou plus à épurer du plasma sanguin. Ce traitement médical est indiqué en cas d'insuffisance rénale chronique terminale.

Les dialysats les plus couramment utilisés sont composés d'une solution tampon (du lactate ou du bicarbonate) à pH acide (5,2 - 5,5) ou physiologique (7,4) à laquelle sont ajoutés des électrolytes (sodium, calcium, magnésium, chlore) et surtout un agent osmotique (du glucose ou un polymère de glucose, tel que l'« icodextrine » présent dans la solution pour dialyse péritonéale ambulatoire EXTRANEAL® commercialisée par la société BAXTER).

Le polymère de glucose, tel l'icodextrine mentionné ci-avant, est préféré au glucose comme agent osmotique, car en raison de sa petite taille, le glucose qui traverse rapidement le péritoine mène à la perte de gradient osmotique dans les 2 à 4 heures d'infusion.

Dans le domaine plus particulier de l'utilisation des polymères du glucose destinés à la dialyse péritonéale continue et ambulatoire, il est très vite apparu que ces hydrolysats d'amidon (mélange de glucose, d'oligomères et de polymères de glucose) ne pouvaient pas être utilisés tels quels.

La demande de brevet européen EP 207.676 enseigne qu'il est préféré des polymères de glucose formant des solutions limpides et incolores à 10 % dans l'eau, ayant un poids moléculaire moyen en poids (Mw) de 5.000 à 100.000 daltons et un poids moléculaire moyen en nombre (Mn) inférieur à 8.000 daltons.

De tels polymères de glucose comprennent aussi de façon préférée au moins 80 % de polymères du glucose dont le poids moléculaire est compris entre 5.000 et 50.000 daltons, peu ou pas de glucose ou de polymères du glucose de DP inférieur ou égal à 3 (poids moléculaire 504) et peu ou pas de polymères de glucose de poids moléculaire supérieur à 100.000 (DP voisin de 600).

En d'autres termes, les polymères de glucose préférés sont des polymères de glucose de faible indice de polymolécularité (valeur obtenue en calculant le rapport Mw/Mn).

Les procédés proposés dans cette demande de brevet EP 207.676 pour obtenir ces polymères de glucose de faible indice de polymolécularité à partir d'hydrolysats d'amidon consistent :
- soit à effectuer une précipitation fractionnée d'une maltodextrine à l'aide d'un solvant miscible à l'eau,
- soit à effectuer une filtration moléculaire de cette même maltodextrine au travers de différentes membranes possédant un seuil de coupure ou d'exclusion adéquat.

Dans les deux cas, ces procédés visent à éliminer à la fois les polymères de très haut poids moléculaire et les monomères ou oligomères de faible poids moléculaire.

Ces procédés ne donnent toutefois pas satisfaction tant du point de vue de leur mise en oeuvre que du point de vue des rendements et de la qualité des produits qu'ils permettent d'obtenir.

Soucieuse de mettre au point un procédé de fabrication d'un polymère de glucose complètement soluble dans l'eau et de faible indice de polymolécularité préférentiellement inférieur à 2,5, ayant de préférence un Mn inférieur à 8.000 daltons et possédant un Mw compris entre 12.000 et 20.000 daltons, procédé qui soit dépourvu des inconvénients de l'art antérieur, la société Demanderesse, s'est attachée à résoudre ce problème dans son brevet EP 667.356, en partant d'un amidon hydrolysé, plutôt que d'une maltodextrine.

Le polymère de glucose obtenu par fractionnement chromatographique contient alors de préférence moins de 3 % de glucose et de polymères de glucose de DP inférieur ou égal à 3 et moins de 0,5 % de polymères de glucose de DP supérieur à 600.

Il est finalement désormais admis par les experts du domaine de la dialyse péritonéale que ces polymères de glucose, utilisés pour leur pouvoir osmotique, donnent toute satisfaction.

Il est cependant à déplorer des risques de contamination microbienne de ces préparations destinées à la dialyse péritonéale.

Il est en effet connu que les circuits de production des polymères de glucose peuvent être contaminés par des microorganismes, ou par des substances pro-inflammatoires contenus dans lesdits microorganismes.

Il est par exemple décrit en amidonnerie la contamination des amidons de maïs ou de blé par des microorganismes de type levures, moisissures et bactéries, et plus particulièrement par des bactéries acidothermophiles de type *Alicyclobacillus acidocaldarius* (bactéries extrémophiles qui se développent dans les zones chaudes et acides du circuit).

Le risque majeur pour le patient qui reçoit ces produits contaminés est alors la péritonite.

Ces épisodes de péritonite sont provoqués par des infections bactériennes intrapéritonéales, et le diagnostic est habituellement facilement établi par les cultures positives de dialysat.

La « péritonite stérile », décrite en tant que péritonite aseptique, chimique, ou culture-négative, est quant à elle typiquement provoquée par un irritant chimique ou un corps étranger.

Depuis l'introduction de l'icodextrine pour la préparation de solutions de dialyse péritonéale, des cas isolés de péritonite aseptique ont été rapportés, pouvant être liés à des causes diverses et notamment l'induction par des substances pro-inflammatoires potentiellement présentes.

Les épisodes inflammatoires aseptiques sont donc des complications majeures observées après injections de solutions de dialyse.

Si une partie de ces épisodes inflammatoires est liée à un problème d'ordre chimique (injection accidentelle de contaminants chimiques ou mauvais dosages de certains composés), la majorité des cas est directement associée à la présence de contaminants d'origine microbienne présents dans les solutions servant à la préparation des solutions de dialyse.

Les lipopolysaccharides (LPS) et les peptidoglycanes (PGN) sont les principaux contaminants d'origine microbienne présentant un risque élevé de déclencher une inflammation même lorsqu'ils sont présents à l'état de traces.

Il est par ailleurs du mérite de la société Demanderesse d'avoir également pris en compte la présence de molécules susceptibles d'exacerber la réponse inflammatoire induite par ces contaminants, tels que les produits de dépolymérisation des PGN, dont la structure minimale encore bioactive est le muramyl-dipeptide (MDP).

Ces dérivés, considérés isolément, sont peu inflammatoires *in vitro* et donnent une réponse significative pour des valeurs supérieures 1 µg/ml.

En plus des produits de dépolymérisation du PGN, les peptides microbiens formylés, dont le prototype est le f-MLP (tripeptide formyl-Met-Leu-Phe), ont également une activité synergique importante. A l'origine, ces peptides ont été identifiés pour leur activité chimio-attractante sur les leucocytes, alors qu'ils sont incapables d'induire une réponse cytokinique *per se.*

Il est donc important de ne pas négliger ces "petites molécules", car elles peuvent rendre compte indirectement des épisodes inflammatoires aseptiques en exacerbant les effets de traces de PGN et/ou de LPS.

La Pharmacopée propose une batterie de test pour la détection de substances pyrogènes :
- Le test de détection des endotoxines bactériennes, composants majoritaires des bactéries Gram négatives (test LAL),
- Le test pyrogène lapin.

Bien que généralement fiables, ces deux tests présentent leurs limites. Le test pyrogène lapin est fondé sur la détection indirecte de substances pyrogènes par la mesure d'une élévation de température du lapin auquel a été injecté le produit contenant ces substances (réponse fébrile).

Ce test peut donner lieu à des faux négatifs, si la substance indésirable présente une activité biologique trop faible ou une concentration trop basse pour induire une réponse systémique pyrogène.

Par ailleurs, cette substance peut posséder une activité biologique ou concentration suffisante pour produire une réaction inflammatoire locale.

Le test LAL quant à lui ne détecte que les endotoxines bactériennes (LPS) ainsi que les β glucanes, composants des parois de flores fongiques. Les autres impuretés biologiques (ADN, peptidoglycanes...) ne sont pas détectées.

La manifestation des péritonites aseptiques observées avec les solutions de dialyse péritonéale contenant de l'icodextrine témoigne donc, pour certains cas, de la façon dont certaines substances peuvent échapper aux tests décrits dans les pharmacopées et peuvent être à l'origine d'effets cliniques indésirables.

Pour remédier à cette situation, la société BAXTER avait proposé de placer les efforts dans la détection des contaminants microbiens Gram positifs.

En particulier, dans son brevet EP 1.720.999 (US2005/191717), la société BAXTER proposa de développer une méthode basée sur la détection des peptidoglycanes, qui sont les composants majeurs des membranes des bactéries Gram positives, notamment dans les polymères de glucose destinés à la préparation de solution pour dialyse péritonéale. WO2007/076411 décrit un test de détection de contaminants pyrogènes reposant sur l'utilisation de monocytes.

En d'autres termes, pour empêcher la survenue de ces épisodes de péritonites aseptiques, la société BAXTER a proposé pour la fabrication et l'usage des solutions de dialyse péritonéale, un protocole de détection de peptidoglycanes dans la solution de dialyse péritonéale.

Par ailleurs, s'il est évoqué dans ce brevet EP 1.720.999 le traitement amont des polymères de glucose, il ne l'est qu'au moyen de résines d'affinités susceptibles de piéger les peptidoglycanes en tant que tels.

Il n'était donc pas envisagé de modifier le procédé de fabrication des polymères de glucose de telle sorte que le produit final soit dépourvu de contamination par des bactéries acidothermophiles de type *Alicyclobacillus acidocaldarius* ou par des débris membranaires de ces bactéries particulières.

Par contre, dans sa demande de brevet internationale WO 2010/125315, la société Demanderesse fournissait, par un procédé de préparation et de purification sécurisé, des substances destinées à la dialyse péritonéale de meilleure qualité, en l'occurrence des polymères de glucose, afin d'assurer que ces substances soient efficacement dépourvues de substances contaminantes.

La société Demanderesse a mis en oeuvre un procédé de purification remarquable, combinant un certain nombre d'étapes de traitement au charbon actif / noir granulaire, de filtration (microfiltration et ultrafiltration) et de traitement thermique dans un agencement propre à empêcher toute contamination.

Cependant, ce procédé est perfectible, et la société Demanderesse s'est employée à développer des méthodes de détection et de dosage plus efficaces que ceux accessibles dans l'état de l'art, afin de mieux définir les étapes clefs de procédé à mettre en oeuvre pour assurer une sécurité optimale des lignes de production, notamment des polymères de glucose.

FR2978774 et WO2012/143647 portent sur l'utilisation de modèles cellulaires pour mesurer la présence de molécules pro-inflammatoires dans des échantillons de polymères de glucose.

De tout ce qui précède, il demeure un besoin non satisfait de fournir, par un procédé de préparation et de purification sécurisés, des substances destinées à des applications thérapeutiques de meilleure qualité, en l'occurrence de polymères de glucose et en ses hydrolysats, afin d'assurer que ces substances soient efficacement dépourvues de contaminants.

La société Demanderesse a donc trouvé que ce besoin pouvait être satisfait par la mise en oeuvre d'étapes de purification adaptées, dont l'efficacité peut être mesurée à l'aide de méthodes de détection et de dosages des contaminants tout à fait particulières.

Ces dernières années, de nombreux tests utilisant des cellules primaires se sont développés pour remplacer les modèles animaux dans les tests de réponse inflammatoire.

Toutefois, ces modèles *in vitro* sont sujets à une importante variabilité interindividuelle, ce qui peut être responsable de biais expérimentaux.

A l'inverse, les lignées cellulaires monocytaires donnent des réponses constantes, ce qui explique pourquoi les tests actuellement en développement utilisent de plus en plus ce type de cellules en culture. Cependant, ces tests présentent l'inconvénient de donner une réponse inflammatoire globale à tous les contaminants présents en mélange dans une solution, et par conséquent ne permettent pas de caractériser la nature du contaminant.

Il est également important de noter que la réponse inflammatoire exacerbée est visible pour les cytokines de la phase aiguë de l'inflammation, telles que TNF-α (Tumor Necrosis Factor alpha), l'IL-1β (interleukine 1β) et les chimiokines telles que CCL5 (Chemokine (C-C motif) ligand 5) /RANTES (Regulated upon Activation, Normal T-cell Expressed, and Secreted), mais pas ou peu pour l'IL-6 (interleukine 6).

Ainsi, les méthodes basées sur la production de cette dernière (US 2009/0239819 et US 2007/0184496) ne sont adaptées pour détecter des contaminants en mélange dans une solution.

La société Demanderesse a abouti aux conclusions suivantes:
*(i)* il est difficile de détecter des contaminants bactériens présents à l'état de traces dans des solutions biologiques,
*(ii)* il est important de ne pas se limiter à la détection des PGN et du LPS, en raison des effets synergiques,
*(iii)* il est nécessaire de développer de nouvelles méthodes de détection sensibles et reproductibles, et
*(iv)* il est avantageux d'utiliser des méthodes de détection sensibles et reproductibles, capables de caractériser la nature des contaminants.

Il a donc été du mérite de la société Demanderesse d'avoir développé des méthodes sensibles et efficaces de détection des contaminants microbiens ayant une action pro-inflammatoire, en deçà du seuil de sensibilité des procédures actuellement utilisées et/ou décrites dans la littérature, et ultérieurement d'avoir identifié la famille, voire la nature, des molécules pro-inflammatoires présentes sous formes de traces dans les lots provenant des circuits de production.

### Résumé de l'invention

La présente invention est relative à un procédé permettant de tester l'effet d'étape(s) de production ou l'efficacité d'étape(s) de purification sur la présence ou la nature des molécules pro-inflammatoires dans des polymères de glucose ou leurs hydrolysats, comprenant :
a) fournir des polymères de glucose ou leurs hydrolysats ;
b) détecter ou doser les molécules pro-inflammatoires dans les polymères de glucose ou leurs hydrolysats fournis à l'étape a) ;
c) effectuer la ou les étapes de production ou de purification sur les polymères de glucose ou leurs hydrolysats fournis à l'étape a) ;
d) détecter ou doser les molécules pro-inflammatoires dans les polymères de glucose ou leurs hydrolysats obtenus après l'étape c) ;
e) déterminer l'efficacité ou l'impact de l'étape c) sur la présence ou la nature des molécules pro-inflammatoires par comparaison des molécules pro-inflammatoires dans les polymères de glucose ou leurs hydrolysats détectées ou dosées aux étapes b) et d), une diminution de la quantité de molécules pro-inflammatoires ou de certaines de ces molécules étant indicative de l'efficacité de l'étape c) pour la décontamination des polymères de glucose ou de leurs hydrolysats;
dans lequel l'étape de détection ou dosage des molécules pro-inflammatoires dans les polymères de glucose ou leurs hydrolysats comprend un test *in vitro* de réponse inflammatoire à l'aide d'une lignée cellulaire exprimant le récepteur TLR2 (Toll Like Receptor 2)) et un gène rapporteur dont la transcription est sous la dépendance directe de la voie de signalisation associée au récepteur TLR2, les molécules pro-inflammatoires étant détectées ou dosées par mesure de l'activité ou du signal du gène rapporteur.

Elle est également relative à un procédé optimisé de production ou de purification de polymères de glucose ou de leurs hydrolysats, comprenant :
a) fournir des polymères de glucose ou leurs hydrolysats ;
b) détecter ou doser les molécules pro-inflammatoires dans les polymères de glucose ou leurs hydrolysats fournis à l'étape a) ;
c) sélectionner la ou les étapes de production ou de purification des polymères de glucose ou de leurs hydrolysats adaptée(s) aux molécules pro-inflammatoires présentes dans les polymères de glucose ou leurs hydrolysats ;
d) effectuer la ou les étapes de production ou de purification sélectionnée(s) sur les polymères de glucose ou leurs hydrolysats fournis à l'étape a) ; et
e) détecter ou doser les molécules pro-inflammatoires dans les polymères de glucose ou leurs hydrolysats obtenus après l'étape d) ;
dans lequel l'étape de détection ou dosage des molécules pro-inflammatoires dans les polymères de glucose ou leurs hydrolysats comprend un test *in vitro* de réponse inflammatoire à l'aide d'une lignée cellulaire exprimant le récepteur TLR2 (Toll Like Receptor 2)et un gène rapporteur dont la transcription est sous la dépendance directe de la voie de signalisation associée au récepteur TLR2, les molécules pro-inflammatoires étant détectées ou dosées par mesure de l'activité ou du signal du gène rapporteur.

Dans un premier aspect, le test *in vitro* de réponse inflammatoire peut comprendre la mise en contact des polymères de glucose ou de leurs hydrolysats avec la lignée cellulaire THP-1 différenciée en macrophage, sensibilisée par MDP ou LPS, les molécules pro-inflammatoires étant détectée ou dosée par mesure de la quantité de RANTES ou TNF-α produite par la lignée cellulaire.

Dans un deuxième aspect, le test *in vitro* de réponse inflammatoire peut comprendre la mise en contact des polymères de glucose ou de leurs hydrolysats avec une lignée de macrophages transfectée avec un gène rapporteur dont la transcription est sous la dépendance directe des voies de signalisation inflammatoires, les molécules pro-inflammatoires étant détectée ou dosée par mesure de l'activité ou du signal du gène rapporteur.

Dans un troisième aspect, le test *in vitro* de réponse inflammatoire peut comprendre la mise en contact des polymères de glucose ou de leurs hydrolysats avec une lignée cellulaire exprimant le récepteur NOD2 et un gène rapporteur dont la transcription est sous la dépendance directe des voies de signalisation de NOD2, les molécules pro-inflammatoires étant détectée ou dosée par mesure de l'activité ou du signal du gène rapporteur.

Dans un quatrième aspect, le test *in vitro* de réponse inflammatoire peut comprendre la mise en contact des polymères de glucose ou de leurs hydrolysats avec une lignée cellulaire exprimant le récepteur TLR4 et un gène rapporteur dont la transcription est sous la dépendance directe des voies de signalisation de TLR4 les molécules pro-inflammatoires étant détectée ou dosée par mesure de l'activité ou du signal du gène rapporteur.

Dans un cinquième aspect, le test *in vitro* de réponse inflammatoire peut comprendre la mise en contact des polymères de glucose ou de leurs hydrolysats avec :
a. la lignée cellulaire THP-1 différenciée en macrophage, sensibilisée par MDP ou LPS, les molécules pro-inflammatoires étant détectée ou dosée par mesure de la quantité de RANTES ou TNF-α produite par la lignée cellulaire ; et,
b. une lignée de macrophages transfectée avec un gène rapporteur dont la transcription est sous la dépendance directe des voies de signalisation inflammatoires, les molécules pro-inflammatoires étant détectée ou dosée par mesure de l'activité ou du signal du gène rapporteur ; et,
c. une lignée cellulaire exprimant le récepteur TLR2 et un gène rapporteur dont la transcription est sous la dépendance directe des voies de signalisation de TLR2, les molécules pro-inflammatoires étant détectée ou dosée par mesure de l'activité ou du signal du gène rapporteur ; et
d. une lignée cellulaire exprimant le récepteur NOD2 et un gène rapporteur dont la transcription est sous la dépendance directe des voies de signalisation de NOD2, les molécules pro-inflammatoires étant détectée ou dosée par mesure de l'activité ou du signal du gène rapporteur ; et,
e. une lignée cellulaire exprimant le récepteur TLR4 et un gène rapporteur dont la transcription est sous la dépendance directe des voies de signalisation de TLR4, les molécules pro-inflammatoires étant détectée ou dosée par mesure de l'activité ou du signal du gène rapporteur.

Dans un sixième aspect, le test *in vitro* de réponse inflammatoire peut comprendre en outre la mise en contact des polymères de glucose ou de leurs hydrolysats avec une lignée contrôle non transfectée par un récepteur de l'immunité.

De préférence, les molécules pro-inflammatoires sont des molécules d'origine bactérienne, de préférence sélectionnées parmi PGN, des LPS, des lipopeptides, des produits de dépolymérisation des PGN, notamment du MDP, les peptides microbiens formylés comme le f-MLP, et des β glucanes.

De préférence, la ou les étapes de production ou de purification sont choisies parmi des étapes de traitement thermique, d'acidification, de passage sur charbon actif, de passage sur des résines d'adsorption, d'ultrafiltration, de filtration, d'hydrolyse chimique ou enzymatique.

De préférence, les polymères de glucose sont sélectionnés parmi de l'icodextrine et des maltodextrines, en particulier des maltodextrines branchées ou non, et les hydrolysats de polymères de glucose sont un produit d'hydrolyse totale tel le dextrose monohydrate.

De préférence, les échantillons de polymères de glucose ou de leurs hydrolysats sont préalablement filtrés, notamment avec un seuil de coupure à 30 kDa, et le filtrat est mis en contact avec la lignée cellulaire utilisée dans le test.

### Description détaillée de l'invention

La présente description est donc relative à un procédé permettant de tester l'impact ou l'effet d'étape(s) de production ou l'efficacité d'étape(s) de purification sur la présence ou la nature des molécules pro-inflammatoires dans des polymères de glucose ou leurs hydrolysats, comprenant :
a) fournir des polymères de glucose ou leurs hydrolysats ;
b) facultativement, détecter ou doser les molécules pro-inflammatoires dans les polymères de glucose ou leurs hydrolysats fournis à l'étape a) ;
c) effectuer la ou les étapes de production ou de purification sur les polymères de glucose ou leurs hydrolysats fournis à l'étape a) ;
d) détecter ou doser les molécules pro-inflammatoires dans les polymères de glucose ou leurs hydrolysats obtenus après l'étape c) ;
e) déterminer l'efficacité ou l'impact de l'étape c) sur la présence ou la nature des molécules pro-inflammatoires ;
dans lequel l'étape de détection ou dosage des molécules pro-inflammatoires dans les polymères de glucose ou leurs hydrolysats comprend un test *in vitro* de réponse inflammatoire à l'aide d'une lignée cellulaire, la lignée cellulaire étant soit un macrophage ou une lignée cellulaire différenciée en macrophage, soit une cellule exprimant un ou plusieurs récepteurs TLR (Toll Like Receptor) ou NOD (*Nucleotide-binding Oligomerization Domain-containing proteins*) tels que TLR2, TLR4 ou NOD2 et permettant de détecter les réponses du ou des récepteurs, soit une combinaison de celles-ci.

La méthode peut comprendre, notamment dans le contexte de l'étape e), une comparaison des molécules pro-inflammatoires dans les polymères de glucose ou leurs hydrolysats détectées ou dosées aux étapes b) et d). Ainsi, une diminution de la quantité des molécules pro-inflammatoires ou de certaines de ces molécules est indicative de l'efficacité d'une étape de production ou de purification pour la décontamination des polymères de glucose ou de leurs hydrolysats. La quantité et la nature des molécules pro-inflammatoires seront déterminées par les méthodes détaillées ci-dessous.

Le but de ce procédé est en particulier la mise au point d'un procédé optimisé de décontamination des polymères de glucose ou de leurs hydrolysats, en particulier les polymères de glucose destinés à la préparation de solution pour dialyse péritonéale, ce procédé comprenant de préférence une détection ou un dosage des molécules pro-inflammatoires un test de réponse inflammatoire *in vitro.*

Notamment, une fois caractérisée l'impact ou l'efficacité des étapes de purification ou production sur les molécules pro-inflammatoires, notamment suivant leur présence et leur nature, l'homme du métier est capable de choisir les étapes convenant le mieux en considération des molécules pro-inflammatoires présentes dans les polymères de glucose ou leurs hydrolysats. Ainsi, le procédé optimisé comprend :
a) fournir des polymères de glucose ou leurs hydrolysats ;
b) détecter ou doser les molécules pro-inflammatoires dans les polymères de glucose ou leurs hydrolysats fournis à l'étape a) ;
c) sélectionner la ou les étapes de production ou de purification des polymères de glucose ou de leurs hydrolysats adaptée(s) aux molécules pro-inflammatoires présentes dans les polymères de glucose ou leurs hydrolysats ;
d) facultativement, effectuer la ou les étapes de production ou de purification sélectionnée(s) sur les polymères de glucose ou leurs hydrolysats fournis à l'étape a) ; et
e) facultativement, détecter ou doser les molécules pro-inflammatoires dans les polymères de glucose ou leurs hydrolysats obtenus après l'étape d) ;
dans lequel l'étape de détection ou dosage des molécules pro-inflammatoires dans les polymères de glucose ou leurs hydrolysats comprend un test *in vitro* de réponse inflammatoire à l'aide d'une lignée cellulaire, la lignée cellulaire étant soit un macrophage ou une lignée cellulaire différenciée en macrophage, soit une cellule exprimant un ou plusieurs récepteurs TLR (Toll Like Receptor) ou NOD (*Nucleotide-binding Oligomerization Domain-containing proteins*) tels que TLR2, TLR4 ou NOD2 et permettant de détecter les réponses du ou des récepteurs, soit une combinaison de celles-ci.

Les polymères de glucose ou leurs hydrolysats peuvent être destinés à la dialyse péritonéale, la nutrition entérale et parentérale, et l'alimentation des nouveaux nés.

Dans un aspect préféré, les polymères de glucose, qui seront préparés dans le cadre de la présente description, sont de l'icodextrine ou des maltodextrines (branchées ou non, comme il sera décrits ci-après).

Les hydrolysats de polymères de glucose dont il est question ici s'entendent notamment du produit d'hydrolyse totale tel le dextrose monohydrate apyrogène, commercialisé sous la marque LYCADEX® PF par la société Demanderesse.

Ils peuvent être décontaminés à un ou plusieurs stades de leur préparation, et notamment au niveau de la matière première, à une étape quelconque de leur procédé de préparation, et/ou au niveau du produit final du procédé.

Ainsi, les polymères de glucose ou leurs hydrolysats fournis dans les procédés selon la présente description correspondent à la matière première, au produit à un niveau quelconque du procédé de préparation ou au produit final.

Les contaminants pro-inflammatoires sont surtout des molécules d'origine bactérienne. Ils peuvent être en particulier des PGN, des LPS, des lipopeptides, des produits de dépolymérisation des PGN, notamment du MDP, les peptides microbiens formylés comme le f-MLP, des β glucanes, etc...

Les méthodes de mesure des réponses inflammatoires *in vitro* qui sont utilisées dans le cadre de la présente description pour suivre l'efficacité des étapes de décontamination des procédés de préparation de polymères de glucose à usage thérapeutique chez l'Homme (*e.g*. solutions de dialyse péritonéale) sont basées sur des tests cellulaires (« *bio-assays* ») utilisant des lignées de type monocytes/macrophages (THP-1, et/ou Raw-Blue™) et des lignées transfectées exprimant un récepteur spécifique de l'immunité naturelle (HEK-Blue™).

La lignée THP-1 (88081201, ECACC) est une lignée humaine pro-monocytaire. Pour les tests de réponse pro-inflammatoire, les cellules sont différenciées en monocytes/ macrophages pendant 3 jours en présence de phorbol ester (PMA).

Pour les tests mis en oeuvre selon la présente description, les macrophages ou cellules différenciées en macrophage, notamment les cellules THP-1 différenciées en macrophages, sont sensibilisées en présence de MDP, en particulier le MDP de S. *aureus.* En effet, le MDP est un faible inducteur inflammatoire, mais il est connu pour agir en synergie avec d'autres molécules inflammatoires. Cette propriété repose sur le fait que ces molécules agissent *via* l'entremise de récepteurs différents de celui du MDP, essentiellement les TLRs. Par conséquent, la présence de MDP va exacerber la réponse inflammatoire induite par les contaminants présents dans les solutions de polymères de glucose ou de leurs hydrolysats, permettant ainsi de détecter de faibles doses de contaminants. De préférence, le MDP est ajouté à l'échantillon à une concentration de plus de 1 µg/mL, de préférence à une concentration comprise entre 1 et 100 µg/mL. Dans un aspect tout particulièrement préféré, le MDP est ajouté à l'échantillon à une concentration de 10 µg/mL.

De manière alternative, les macrophages ou cellules différenciées en macrophage, notamment les cellules THP-1 différenciées en macrophages, peuvent être sensibilisées en présence d'autres molécules que le MDP. En effet, le LPS, en particulier un LPS d'*E*. *coli,* peut également être utilisé. Il peut être ajouté à l'échantillon à une concentration d'au moins 10 pg/ml, par exemple à une concentration de 25 pg/mL.

Dans un aspect préféré, les macrophages ou cellules différenciées en macrophage, notamment les cellules THP-1 différenciées en macrophages, sont mis en oeuvre à une densité comprise entre 0,5 à 1 x 10⁶ cellules/mL de milieu de culture, de préférence comprise entre 0,7 à 0,8 x 10⁶ cellules/mL, et de manière encore plus préférée environ 0,75 x 10⁶ cellules/mL.

Le test de réponse inflammatoire *in vitro* est basé sur la mesure de la production de RANTES par les cellules THP-1 sensibilisées. En effet, les études antérieures ont montré que le dosage de cette chimiokine est adapté pour détecter de faibles doses de contaminants, notamment les endotoxines, dans les solutions de polymères de glucose. De manière alternative, le test de réponse inflammatoire *in vitro* peut également être basé sur la mesure de la production de TNF-α par les cellules THP-1 sensibilisées. Les dosages des cytokines peuvent être faits par tout moyen bien connu de l'homme du métier, et notamment par ELISA. Dans un aspect préféré, le test comprend la mesure de la production de TNF-α après 8 h de stimulation. Dans un autre aspect préféré, le test comprend la mesure de la production de RANTES après 20 h de stimulation, notamment par un dosage ELISA.

Ce premier test permet de détecter en particulier la contamination des polymères de glucose ou de leurs hydrolysats par des PGN et/ou LPS, de préférence par des PGN de taille moyenne (notamment d'environ 120 kDa) et/ou LPS, plus particulièrement encore par des LPS.

La lignée Raw-Blue™ est une lignée de macrophages de souris transfectés avec un gène rapporteur produisant une forme sécrétée de la phosphatase alcaline (SEAP: *secreted embryonic alkaline phosphatase*), dont la transcription est sous la dépendance directe des voies de signalisation inflammatoires. L'avantage de cette lignée est qu'elle exprime de façon naturelle la quasi-totalité des récepteurs de l'immunité innée, dont les récepteurs TLR2, TLR4 et NOD2. Ainsi, ces cellules vont répondre à la majorité des contaminants inflammatoires, et la réponse sera suivie par mesure de l'activité enzymatique de la SEAP produite. De préférence, cette lignée est utilisée dans le test à une densité cellulaire d'environ 0,5 x 10⁶ cellules/puits. La mise en contact de la préparation de polymères de glucose ou de leurs hydrolysats avec les cellules dure environ 16 à 24 h.

Les lignées cellulaires, notamment HEK-Blue™ (InvivoGen), sont des lignées modifiées par transfection stable avec un vecteur codant pour un récepteur de l'immunité innée, en particulier des récepteurs humains (h). Elles sont également co-transfectées avec le gène rapporteur, notamment un gène rapporteur produisant la SEAP, dont la synthèse est sous la dépendance directe de la voie de signalisation associée au récepteur surexprimé. De manière préférée, ce gène rapporteur code pour une protéine colorée ou fluorescente ou pour une protéine dont l'activité peut être mesurée avec ou sans substrat. La détection de l'activité ou du signal du gène rapporteur indique que l'échantillon contient des contaminants susceptibles d'activer un ou des récepteurs de l'immunité innée et de déclencher une réaction inflammatoire. L'utilisation de ces lignées permet de cibler certaines familles de molécules d'origine microbienne en fonction du récepteur exprimé. De préférence, des lignées cellulaires exprimant soit hTLR2, soit hTLR4, soit hNOD2 sont utilisées. En outre, une lignée contrôle n'exprimant aucun récepteur de l'immunité innée est également utilisée. L'utilisation de cette lignée contrôle est utile pour vérifier que les solutions de polymères de glucose ou de leurs hydrolysats n'induisent pas la production du gène rapporteur par un mécanisme parasite, comme un mécanisme de toxicité.

Pour les tests selon la présente description, quatre lignées sont de préférence utilisées :
- lignée HEK-Blue™ hTLR2 : cette lignée exprimant le récepteur hTLR2 répond spécifiquement aux agonistes du TLR2 (PGN et lipopeptides surtout). Son utilisation permet donc de connaître le taux de ces contaminants dans le déclenchement des réponses inflammatoires,
- lignée HEK-Blue™ hTLR4 : cette lignée exprimant le récepteur hTLR4 répond spécifiquement aux LPS. Son utilisation permet donc de connaître le taux de ces contaminants dans le déclenchement des réponses inflammatoires,
- lignée HEK-Blue™ hNOD2 : cette lignée exprimant le récepteur hNOD2 répond spécifiquement aux agonistes de NOD2. Son utilisation permet donc de connaître le taux de MDP et molécules apparentées dans le déclenchement des réponses inflammatoires,
- lignée HEK-Blue™ Null2: il s'agit d'une lignée contrôle, non transfectée par un récepteur de l'immunité. Son utilisation est nécessaire pour vérifier que les solutions de polymères de glucose ou de leurs hydrolysats n'induisent pas la production de la SEAP par un mécanisme de toxicité.

Cependant, il est à noter que l'homme du métier peut également utiliser d'autres lignées commerciales (Imgenex) ou il peut en préparer.

Dans un aspect préféré, les lignées cellulaires sont mises en oeuvre à une densité comprise entre 0,5 à 1 x 10⁶ cellules/mL de milieu de culture, et la mise en contact de la préparation de polymères de glucose ou leurs hydrolysats avec les cellules dure environ 16 à 24 h .

Une quantification des contaminants peut être réalisée à l'aide d'une courbe dose-réponse. Cette courbe dose-réponse peut notamment être réalisée avec les mêmes cellules, dans les mêmes conditions, avec des doses croissantes de contaminants. Les courbes doses-réponses sont en particulier réalisées avec des standards de LPS, PGN, lipopeptide, β-glucanes et MDP. De préférence, une telle courbe dose-réponse peut être réalisée pour les cellules exprimant TLR4 (par exemple, THP-1, HEK-Blue™ hTLR4 et Raw-Blue™) avec des doses croissantes de LPS, pour les cellules exprimant TLR2 (par exemple, THP-1, HEK-Blue™ hTLR2 et Raw-Blue™) avec des doses croissantes de PGN, et pour les cellules réactives via NOD2 (par exemple, HEK-Blue™ hNOD2) avec des doses croissantes de MDP.

Dans des aspects particuliers, les lignées THP-1, Raw-Blue™ et HEK-Blue™ sont incubées avec des concentrations croissantes en standards, et la réponse cellulaire est mesurée par quantification de la production de RANTES par ELISA pour la lignée THP-1 et mesure du gène rapporteur, notamment de l'activité enzymatique de la SEAP pour les lignées Raw-Blue™ et HEK-Blue™.

Le test selon la présente description permet d'identifier le(s) contaminant(s) susceptible(s) de déclencher une réaction inflammatoire. Ainsi, la lignée exprimant NOD2, en particulier HEK-Blue™ hNOD2, permet tout particulièrement de détecter une contamination par des produits de dépolymérisation du PGN et le MDP, de préférence le MDP. La lignée exprimant TLR2, en particulier HEK-Blue™ hTLR2 et/ou Raw-Blue™, permet tout particulièrement de détecter une contamination par des PGN. Par ailleurs, les macrophages, en particulier les macrophages de THP-1, et la lignée exprimant TLR4, en particulier HEK-Blue™ hTLR4, permet tout particulièrement de détecter une contamination par des LPS.

Dans un aspect préféré, le test *in vitro* de réponse inflammatoire inclut des tests avec les lignées cellulaires suivantes :
- les macrophages, en particulier les macrophages de THP-1, une lignée cellulaire permettant de détecter l'activité d'un récepteur TLR2, notamment la lignée HEK-Blue™ hTLR2, une lignée cellulaire permettant de détecter l'activité d'un récepteur TLR4, notamment la lignée HEK-Blue™ hTLR4, une lignée cellulaire permettant de détecter l'activité d'un récepteur NOD2, notamment la lignée HEK-Blue™ hNOD2, et facultativement mais de préférence une lignée contrôle, notamment la lignée HEK-Blue™ Null2 ; ou
- une lignée de macrophages transfectés avec un gène rapporteur, notamment la lignée Raw-Blue™, une lignée cellulaire permettant de détecter l'activité d'un récepteur TLR2, notamment la lignée HEK-Blue™ hTLR2, une lignée cellulaire permettant de détecter l'activité d'un récepteur TLR4, notamment la lignée HEK-Blue™ hTLR4, une lignée cellulaire permettant de détecter l'activité d'un récepteur NOD2, notamment la lignée HEK-Blue™ hNOD2, et facultativement mais de préférence une lignée contrôle, notamment la lignée HEK-Blue™ Null2.

Dans le aspect préféré, la présence de contaminants dans les différents échantillons est testée à l'aide des cinq types cellulaires présentés ci-avant, de façon à avoir un aperçu de la réponse inflammatoire générale, ainsi que des réponses spécifiques à certains contaminants :
- Lignée THP-1 sensibilisée par le MDP : tous contaminants avec forte réactivité pour les LPS,
- Lignée Raw-BlueTM : tous contaminants avec forte réactivité pour les PGN,
- Lignée HEK-BlueTM hTLR2 PGN et autres ligands de TLR2 (lipopeptides...),
- Lignée HEK-BlueTM hTLR4 : LPS,
- Lignée HEK-BlueTM hNOD2 : MDP et produits de dépolymérisation des PGN,
- Lignée HEK-BlueTM Null2 : témoin négatif.

Dans le procédé selon la présente description, la ou les étapes de production ou de purification peuvent être choisies parmi des étapes de traitement thermique, d'acidification, de passage sur charbon actif, de passage sur des résines d'adsorption, d'ultrafiltration, de filtration, d'hydrolyse chimique ou enzymatique, ou des combinaisons de celles-ci. Différents paramètres peuvent être testés pour chaque étape, permettant de sélectionner les plus efficaces. Par exemple, dans le cas d'un passage sur charbon actif, différentes qualités de charbon actif et combinaisons de celles-ci peuvent être testées. Dans le cas d'une ultrafiltration, on pourra tester différents seuils de coupure et/ou les combiner. Dans le cas d'un traitement thermique, on pourra faire varier la température et la durée de traitement. Dans le cas d'un traitement enzymatique, on pourra faire varier la ou les enzymes utilisées, leur concentration et conditions de traitement.

Dans un aspect très particulier, les traitements sont réalisés sur des échantillons préparés à 32 % (poids/volume) dans de l'eau apyrogène (*p.p.i.*), puis les solutions sont filtrées sur 0,22 µm. Pour les tests cellulaires, les échantillons sont dilués au 1/10 dans le milieu de culture des cellules (concentration finale : 3,2 % (p/v)).

Les échantillons de polymères de glucose ou leurs hydrolysats peuvent être en outre soumis à des traitements enzymatiques ou chimiques ou à des étapes de filtration préalablement au test de détection ou dosage des molécules pro-inflammatoires. Facultativement, les résultats obtenus avant et après ces étapes de traitement ou de filtration peuvent être comparés.

Ainsi, un échantillon de polymères de glucose ou de leurs hydrolysats peut être traité par une mutanolysine préalablement au test. Cette enzyme, par son activité muramidase, est capable de dépolymériser les PGN. Par exemple, l'enzyme à une concentration d'environ 2500 U/ml peut être mise en présence de l'échantillon, éventuellement dilué pour avoir une concentration en polymère de glucose de 7,5 à 37,5 % (poids/volume), pendant 6 à 16 h, de préférence environ 16 h. Ensuite, l'échantillon ainsi traité sera soumis au test avec une ou des lignée(s) cellulaire(s) selon la présente description.

Alternativement, l'échantillon de la préparation de polymères de glucose ou de leurs hydrolysats peut être filtré préalablement au test. Le but de cette filtration est essentiellement d'enlever les molécules de haut poids moléculaire, comme les PGN de haut poids moléculaire, et de procéder au test sur le filtrat pour analyser tout particulièrement les contaminants de petites tailles. Le seuil de coupure pour la filtration peut par exemple être compris entre 30 kD et 150 kD, de préférence entre 30 et 100 kD ou entre 30 et 50 kD, et notamment d'environ 30 kD. Dans un aspect préféré, les tests cellulaires sont réalisés sur les fractions obtenues par ultra-filtration, notamment avec des seuils de coupure de 30 et 100 kDa. De préférence, la filtration est faite par une ultrafiltration. Elle peut également être réalisée par tout moyen connu de l'homme du métier. Ainsi, l'échantillon ainsi filtré, le filtrat, sera soumis aux tests cellulaires selon la présente description. La comparaison des résultats obtenus sans ou avant filtration permettra de déduire la contribution inflammatoire spécifique des molécules de petites tailles. Par ailleurs, cela permet de vérifier si les étapes de production ou de purification modifient la taille des contaminants (hydrolyse *versus* agrégation), et/ou n'éliminent pas certains contaminants de taille définie.

Par ailleurs, le traitement des échantillons par le lysozyme et/ou la β-glucanase permet d'éliminer le PGN et/ou les β-glucanes, et de connaître ainsi l'importance des autres agonistes du TLR2 susceptibles d'être présents dans les lots contaminés (glycolipides et lipopeptides).

Dans un aspect préféré des procédés selon la présente description, une première série de tests cellulaires est réalisée sur des échantillons non filtrées, de façon à mesurer les réponses sans tenir compte de la taille des molécules et à conserver les possibles effets synergiques entre ces molécules. Puis dans une seconde série, les tests cellulaires sont réalisés sur les fractions obtenues par ultra-filtration (seuils de coupure : 30 et 100 kDa), de façon à vérifier si les traitements modifient la taille des contaminants (hydrolyse *versus* agrégation), et/ou n'éliminent pas certains contaminants de taille définie.

Pour illustrer le procédé de l'invention, différentes étapes de décontamination sont réalisées sur différentes matrices de polymères de glucose distinctes et un lot d'hydrolysat de polymère de glucose :
- des polymères de glucose, matières premières de l'Icodextrine (avant fractionnement chromatographique selon l'enseignement du brevet EP 667.356),
- un lot d'icodextrine,
- un lot de maltodextrine branchée, commercialisé par la société Demanderesse sous le nom de marque NUTRIOSE® FB06,
- un lot de dextrose monohydrate préparé pour être conditionné en solution injectable, commercialisé par la société Demanderesse sous le nom de marque LYCADEX® PF,
- un lot de polymères solubles de glucose hautement branchés préparé selon l'enseignement de la demande de brevet internationale WO 2007/099212 dont la société Demanderesse est titulaire,
- Une maltodextrine commerciale.

Les traitements retenus sont :
- thermique, par exemple :
   ∘ 70°C et/ou
   ∘ 120°C,
- acidification,
- passage sur charbon actif de diverses qualités, par exemple :
   ∘ SX+ de la société NORIT
   ∘ SX2 de la société NORIT,
   ∘ C EXTRA USP de la société NORIT,
   ∘ A SUPRA EUR de la société NORIT,
   ∘ ENO-PC de la société CECA,
   ∘ L4S de la société CECA,
   ∘ L3S de la société CECA,
   ∘ CSA de la société CECA,
- passage sur résine d'adsorption,
   ∘ Amberlite XAD-4 de la société Rohm & Haas,
   ∘ Amberlite XAD-761 de la société Rohm & Haas,
   ∘ Amberlite XAD-1600 de la société Rohm & Haas,
   ∘ Amberlite XAD-16 HP (= FPX66) de la société Dow,
   ∘ Dowex SD2 de la société Dow,
   ∘ Macronet MN-100 de la société Purolite,
   ∘ Macronet MN-150 de la société Purolite,
- ultrafiltration présentant différent seuil de coupure, par exemple
   - 5 kDa ;
   - 30 kDa ;
- hydrolyse enzymatique à l'aide d'enzymes spécifiques, par exemple :
   ∘ β 1-3 glucanase
   ∘ protéases.
   ∘ préparations enzymatiques à activité mannanase (par exemple la Mannaway®, commercialisée par la société NOVOZYMES, utilisée pour ses propriétés détergentes et de clarification).
   ∘ préparations enzymatiques à activité endo-beta-glucanase (par exemple la SEBflo® TL, produite par la société Specialty Enzymes and Biotechnologies Co. et commercialisée par Advances Enzymes Technologies Ltd.)
   ∘ préparations enzymatiques à activité protéase acide (par exemple la SEBPro® FL100, produite par la société Specialty Enzymes and Biotechnologies Co. et commercialisée par Advances Enzymes Technologies Ltd.).

L'invention sera mieux comprise à l'aide des exemples qui suivent, lesquels se veulent illustratifs.

### Brève description des figures

Figure 1 : Réponses des cellules Raw-Blue™ aux agonistes standards.
Figure 2 : Réponses des cellules HEK-Blue™ TLR2 aux agonistes standards.
Figure 3 : Réponses des cellules HEK-Blue™ TLR4 aux agonistes standards.
Figure 4 : Réponses des cellules HEK-Blue™ NOD2 aux agonistes standards.
Figure 5 : Réponses des cellules HEK-Blue™ Null aux agonistes standards.
Figure 6: Réponses des cellules Raw-Blue™ induites par les matrices non filtrées et après passage sur filtres 100 kDa et 30 kDa.
Figure 7 : Réponses des cellules HEK-Blue™ TLR2 induites par les matrices non filtrées et après passage sur filtres 100 kDa et 30 kDa.
Figure 8 : Réponses des cellules HEK-Blue™ TLR4 induites par les matrices non filtrées et après passage sur filtres 100 kDa et 30 kDa.
Figure 9 : Réponses des cellules HEK-Blue™ NOD2 induites par les matrices non filtrées et après passage sur filtres 100 kDa et 30 kDa.
Figure 10 : Réponses des cellules HEK-Blue™ Null induites par les matrices.
Figure 11 : Bilan des activités inflammatoires des différentes matrices
Figure 12: Réponses cellulaires induites par les matrices après passage sur charbon SX+.
Figure 13 : Réponses cellulaires induites par la matrice E3063 après passage sur différents charbons.
Figure 14 : Réponses cellulaires induites par la matrice E1565 après passage sur différents charbons.
Figure 15 : Réponses cellulaires induites par la matrice E1242 après passage sur différents charbons.
Figure 16 : Réponses cellulaires induites par la matrice Lab3943 après passage sur différents charbons.
Figure 17 : Réponses cellulaires induites par la matrice E1565 après traitement par ultrafiltration sur 5 kDa.
Figure 18 : Réponses cellulaires induites par la matrice Lab3943 après traitement par ultrafiltration sur 5 kDa.
Figure 19 : Réponses cellulaires induites par les matrices E3063 et E5250 après traitement par la Mannaway®.
Figure 20 : Réponses cellulaires induites par la matrice E5250 après traitement par la SEBflo®TL.
Figure 21 : Réponses cellulaires induites par la matrice E30630 après traitement par la SEBPro® FL100.
Figure 22 : Réponses cellulaires induites par la matrice E1565 après traitement par des résines industrielles.

### Exemples

### Exemple 1 : Etablissement des courbes doses-réponses

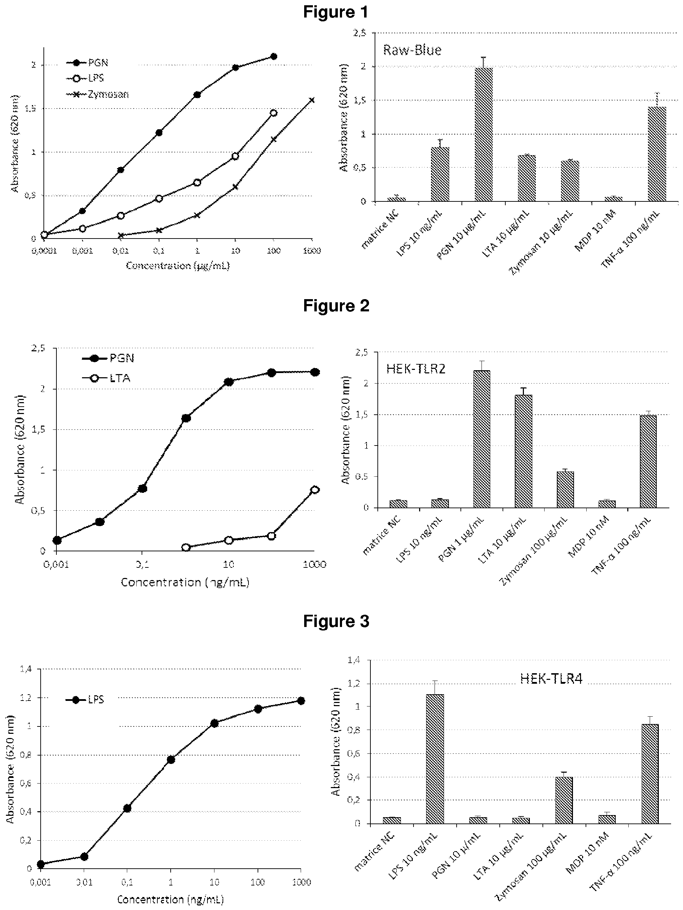

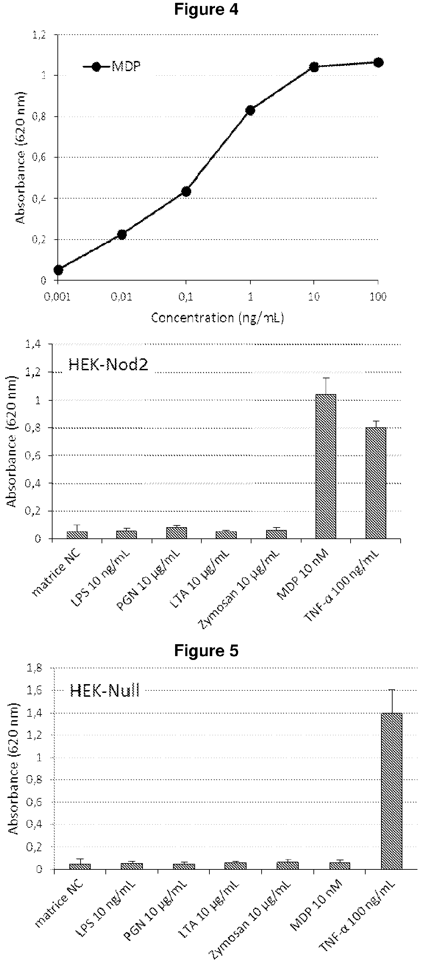

Les courbes doses-réponses sont réalisées avec des molécules agonistes standards : LPS, PGN, LTA, zymosan et MDP. Les lignées Raw-Blue™ et HEK-Blue™ TLR2, TLR4, NOD2 et Null sont incubées avec des concentrations croissantes en agonistes, et la réponse cellulaire est mesurée par quantification de l'activité SEAP (Figures 1-5). Le TNF-α est utilisé comme témoin positif d'activation des cellules :
- Lignée Raw-Blue™ : les cellules répondent aux molécules inflammatoires majeures susceptibles d'être présentes dans les matrices et dérivés de polymères de glucose (PGN, LPS, zymosan, LTA) ; elles ont notamment une forte réactivité vis à vis des PGN, mais ne répondent pas à leurs produits de dépolymérisation (MDP).
- Lignée HEK-Blue™ hTLR2 : forte réactivité vis-à-vis des PGN ; les cellules répondent plus faiblement aux autres ligands de TLR2 (LTA, zymosan) et ne montrent aucune réactivité vis-à-vis des LPS et du MDP,
- Lignée HEK-Blue™ hTLR4 : forte réactivité vis-à-vis des LPS ; les cellules répondent très faiblement au zymosan et ne montrent aucune réactivité vis-à-vis des PGN, LTA et MDP,
- Lignée HEK-Blue™ hNOD2 : forte réactivité vis-à-vis du MDP,
- Lignée HEK-Blue™ Null2 : témoin d'absence de toxicité cellulaire.

### Exemple 2 : Préparation des différentes matrices de polymère de glucose distinctes et d'un lot d'hydrolysat de polymère de glucose

Comme indiqué ci-avant, les matrices sont les suivantes :
- 5 polymères de glucose, matières premières de l'Icodextrine (avant fractionnement chromatographique selon l'enseignement du brevet EP 667.356) référencés ici E1565, E3063, E1242, E5248 et E5250.

La préparation de ces cinq polymères est réalisée conformément aux enseignements de la demande de brevet WO 2012/059685.
- un lot d'Icodextrine contaminé (référencé ici E209J) et un lot d'icodextrine "standard", i.e. témoin de non-contamination dans les tests cellulaires (référencée ici P11-11). Ces lots sont préparés selon l'enseignement du brevet EP 667.356, détaillé dans l'exemple 1 de la demande de brevet WO 2010/125315.
- un lot de maltodextrine branchée, commercialisé par la société Demanderesse sous le nom de marque NUTRIOSE® FB06.
- un lot de dextrose monohydrate préparé pour être conditionné en solution injectable, commercialisé par la société Demanderesse sous le nom de marque LYCADEX® PF,
- un lot de polymères solubles de glucose hautement branchés destiné à la dialyse péritonéale, référencé ici LAB3943.

Ce lot est préparé par le double traitement enzymatique enzyme de branchement et amyloglucosidase selon l'exemple 2 de la demande de brevet WO 2007/099212.
- Une maltodextrine commerciale (Maltodextrine Cargill, C*Dry MD 01915, lot 02044770), référencée ici Cargill.

### Exemple 3 : Analyse des réponses cellulaires induites par les échantillons non traités ou après passage sur filtre 100 kDa ou 30 kDa

L'objectif de ces essais est de déterminer la réactivité pro-inflammatoire et la nature des contaminants présents dans les matrices de polymère de glucose et le lot d'hydrolysat de polymère de glucose.

Les échantillons selon l'exemple 2 sont préparés à 32 % (poids/volume) dans de l'eau apyrogène (*p.p.i*.).

Les dosages des taux LPS et de PGN ont été réalisés préalablement aux tests cellulaires en utilisant les dosages SLP-HS et LAL (données présentées ci-dessous) :

| | | P11-11 | E1242 | E1565 | E3063 | E5250 | Lab 3943 | Ico E209J | Cargill | NUTRIOSE® | LYCADEX® |
|---|---|---|---|---|---|---|---|---|---|---|---|
| PGN (ng/g) | SLP -HS | < 3 | 21 | 2320 | 16185 | 4496 | 1263 | 393 | 2478 | 315 | < 2 |
| LPS (EU/g) | LAL | < 0,3 | 2,4 | 38,4 | 2,4 | 19,2 | 153,6 | 0,6 | 9,6 | > 300 | < 0,15 |
| LPS (EU/g) | LAL modifié | < 0,3 | 1,2 | 4,8 | 1,2 | < 0,3 | 153,6 | < 0,3 | < 0,3 | > 300 | / |

Pour les tests cellulaires, les échantillons sont dilués au 1/10 dans le milieu de culture des cellules (concentration finale : 3,2 % (p/v)).

Les analyses sont réalisées sur :
- Lignée Raw-Blue™ : tous contaminants avec réactivité élevée pour les PGN,
- Lignée HEK-Blue™ hTLR2 : réactivité élevée pour les PGN,
- Lignée HEK-Blue™ hTLR4 : réactivité élevée pour les LPS,
- Lignée HEK-Blue™ hNOD2 : MDP et produits de dépolymérisation des PGN,
- Lignée HEK-Blue™ Null2 : témoin d'absence de toxicité cellulaire.

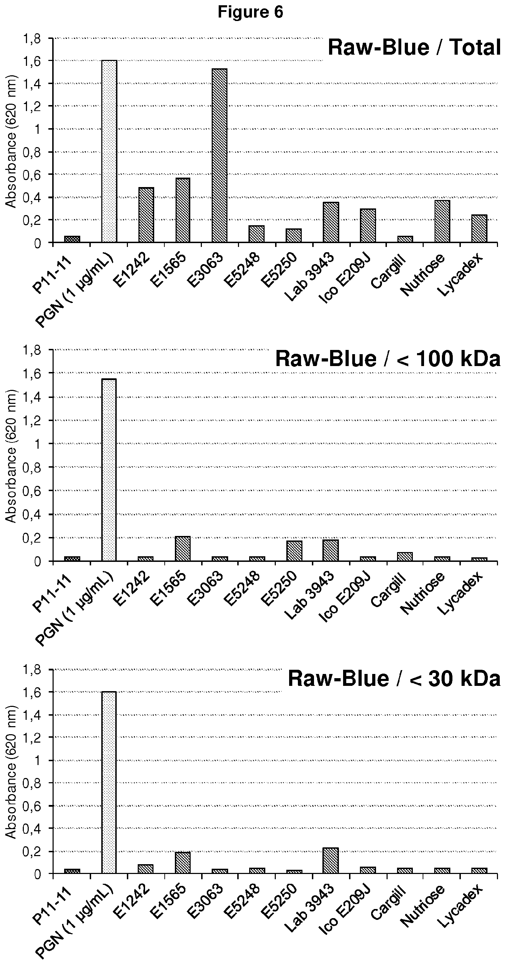

Les résultats par type cellulaire sont présentés dans les figures 6 à 10.

### Réponses des cellules Raws (Figure 6) :

A l'exception de la matrice Cargill, qui donne une réponse équivalente à celle observée en présence du témoin de non-contamination P11-11, tous les autres échantillons déclenchent une réponse inflammatoire au contact de la lignée macrophage. Les matrices les plus réactives sont E-3063 (saturation de la réponse des cellules), puis E-1242 et E-1565.

Les contaminants sont essentiellement des molécules de haut poids moléculaire (par exemple, PGN, zymosan) ou capables de former des agrégats (par exemple, LPS, LTA). En effet, la filtration à 100 kDa réduit fortement les réponses induites par les échantillons, indiquant que ce traitement les a éliminés en grande partie. Seules les matrices E-1242 et E-5250 ont encore une activité significativement supérieure à celle de P11-11, indiquant qu'elles contiennent des contaminants de taille < 100 kDa, provenant probablement de la dégradation de plus gros contaminants. La filtration à 30 kda est encore plus efficace car les différents échantillons perdent la quasi-totalité de leur activité pro-inflammatoire après traitement.

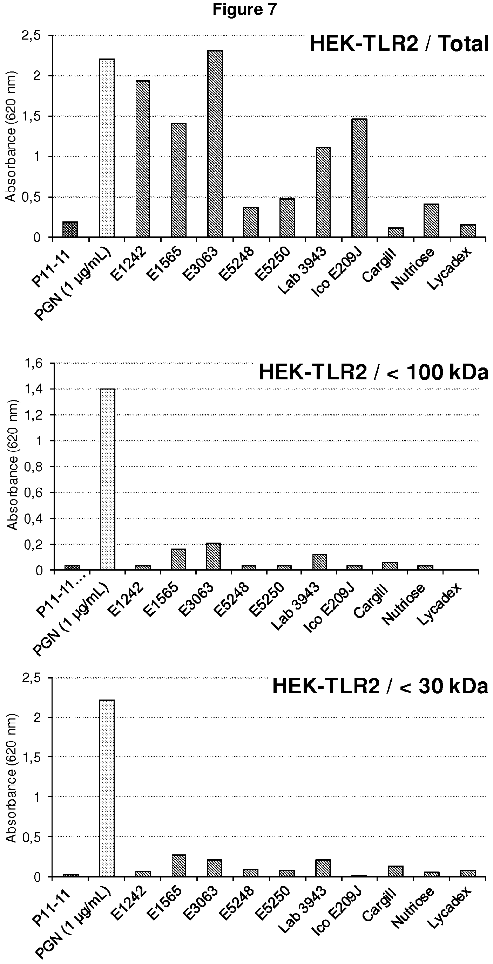

### Réponses des cellules HEK-TLR2 (Figure 7) :

Les résultats obtenus avec les cellules HEK-TLR2 confirment les résultats précédents.

La matrice E-3063 induit une réponse saturée, ce qui indique un taux de contamination très élevé en inducteurs de TLR2. Les matrices E-1242, E-1565, Lab3943 et Ico-E209J donnent également des réponses élevées, plus importantes que celles observées dans les cellules Raws. Cette différence s'explique par le fait qu'elles sont chargées en inducteurs forts de TLR2 (PGN ou lipopeptides).

Les filtrations à 100 kDa et à 30 kDa neutralisent les réponses inflammatoires induites par ces échantillons, indiquant que les contaminants sont majoritairement des PGN de haut poids moléculaire. Toutefois, les matrices E-3063 et E-1565 présentent encore une activité significative après filtration. Ces données montrent que ces composés contiennent des produits de dégradation du PGN et/ou des lipopeptides. En effet, contrairement aux PGN, les autres inducteurs forts de TLR2 ont une masse < 30 kDa et donneraient donc encore une réponse cellulaire après filtration.

Les matrices E-5250, E-5248 et le NUTRIOSE® donnent des réponses faibles, d'intensité équivalente à celle observée avec les cellules Raws, suggérant que ces trois échantillons contiennent des inducteurs faibles de TLR2 (par exemple, zymosan, β-glucanes ou LTA).

Comme précédemment, la matrice Cargill et le LYCADEX® ne déclenchent pas de réponses, indiquant l'absence de contaminants inducteurs de TLR2.

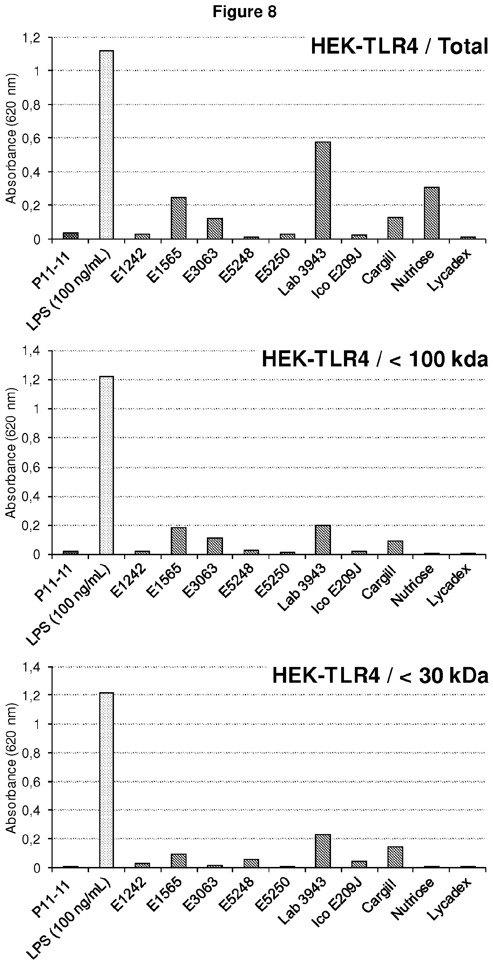

### Réponses des cellules HEK-TLR4 (Figure 8) :

Les matrices E1565, E3063, Lab3943, Cargill et NUTRIOSE® déclenchent une réponse d'intensité moyenne dans les cellules HEK-TLR4, ce qui confirme la présence de LPS. Les filtrations réduisent en partie les réponses des cellules, ce qui s'explique par le fait que le LPS peut former des agrégats, et que seules les molécules non agrégées ont été éliminées.

Les matrices E1242, IcoE209J, E5248, E5250 et le LYCADEX® ne déclenchent pas de réponse significative, indiquant que les taux de LPS sont en-deçà des seuils susceptibles de déclencher une réponse inflammatoire. Les deux premières matrices donnent une réponse inflammatoire dans les cellules Raws, ce qui est corrélée à une forte réactivité dans les cellules HEK-TLR2. Ces données indiquent que ces deux matrices sont essentiellement contaminées par des PGN. Les matrices E5248, E5250 et le LYCADEX® déclenchent également une réponse inflammatoire dans les cellules Raws. Toutefois, elles ne sont que peu ou pas actives vis à vis de TLR2, ce qui montre la présence de contaminants autres que les PGN et les LPS dans ces trois échantillons.

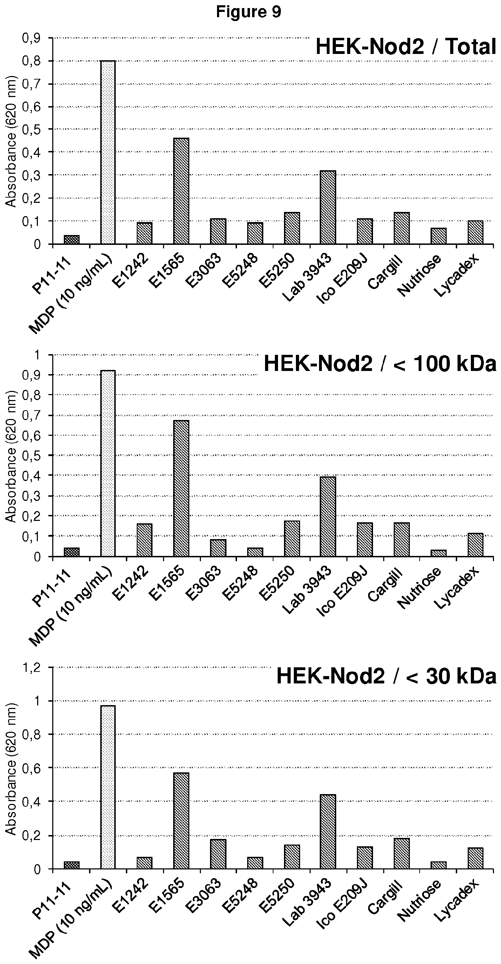

### Réponses des cellules HEK-NOD2 (Figure 9) :

Les cellules HEK-NOD2 répondent à tous les échantillons, mais seuls les matrices E-1565, Lab3943 et Cargill sont fortement chargées en inducteurs de NOD2. Ce récepteur réagit au produit final de dépolymérisation des PGN (MDP), mais également à leurs produits de dégradation de faible masse moléculaire. Par conséquent, l'intensité des réponses observées indiquent que les échantillons sont et/ou ont été contaminés par des PGN ayant subi un processus de dégradation plus ou moins avancé. Comme attendu, les filtrations à 100 et 30 kDa n'ont pas d'effet significatif sur la réponse des cellules, puisque les composés incriminés (MDP et PGN dégradés) sont de petite taille.

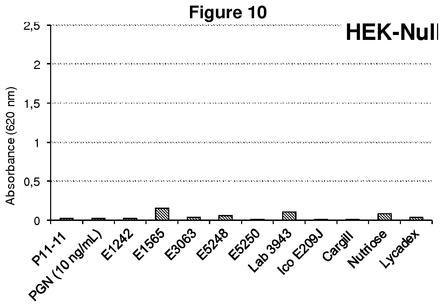

### Réponses des cellules HEK-Null (Figure 10) :

Finalement, les cellules HEK-Null ne donnent pas de réponses significatives en présence des différents échantillons, preuve que les réactivités observées dans les autres lignées cellulaires ne sont pas liées à un effet toxique, mais bien à une réponse de type inflammatoire.

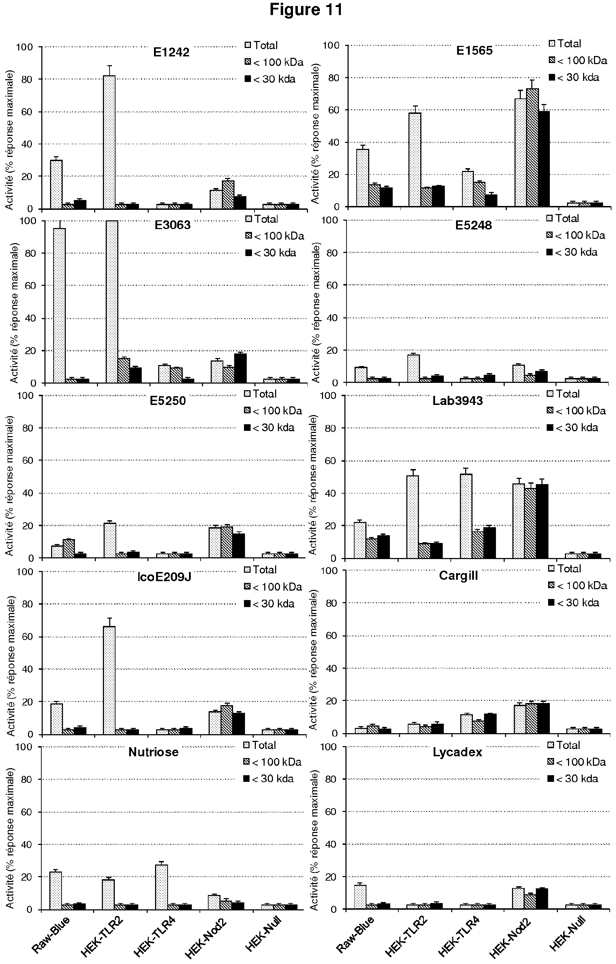

### Bilan par échantillon (Figure 11) :

- E1242: activité inflammatoire d'intensité moyenne, liée à une forte contamination en PGN faiblement dégradés.
- E1565: activité inflammatoire d'intensité moyenne, liée à une forte contamination en PGN partiellement dégradés et à la présence de LPS.
- E3063 : activité inflammatoire d'intensité élevée, liée à une très forte contamination en PGN faiblement dégradés et à des traces de LPS.
- E5248 : activité inflammatoire de faible intensité, liée à une faible contamination en PGN et à la présence de molécules inflammatoires autres que PGN et LPS.
- E5250 : activité inflammatoire de faible intensité, liée à une faible contamination en PGN et à la présence de molécules inflammatoires autres que PGN et LPS.
- Lab3943 : activité inflammatoire d'intensité moyenne, liée à des contaminations moyennes en PGN partiellement dégradés et en LPS.
- IcoE209J : activité inflammatoire d'intensité moyenne, liée à une forte contamination en PGN faiblement dégradé.
- Cargill : absence d'activité inflammatoire détectable, mais présence de produits de dégradation des PGN.
- NUTRIOSE®: activité inflammatoire d'intensité moyenne, liée à des traces de PGN partiellement dégradés et à une contamination moyenne en LPS
- LYCADEX® : activité inflammatoire de faible intensité, liée à une faible contamination en produits de dégradation des PGN et à la présence de molécules inflammatoires autres que PGN et LPS.

### Exemple 4 : Effet des traitements par passage sur charbons actifs.

Dans une première série d'expériences, tous les échantillons ont été soumis à deux traitements successifs avec le même charbon (1 % de charbon NORIT SX+, à 80°C pendant 1 h).

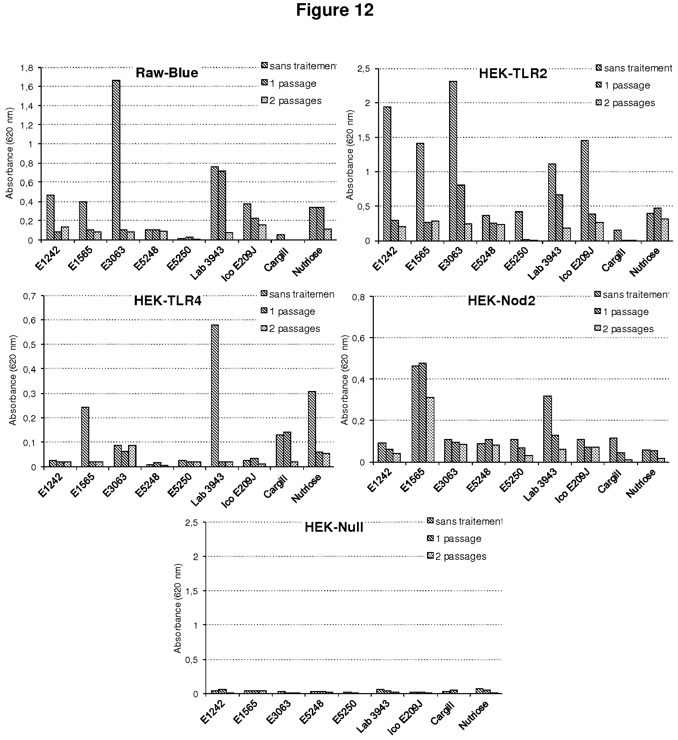

La figure 12 présente les résultats obtenus par lignée cellulaire.

Le premier traitement au charbon diminue de façon drastique la capacité des échantillons E1242, E-565, E3063 et IcoE209J à déclencher une réponse inflammatoire dans les cellules Raws et HEK-TLR2. Dans tous les cas, le second traitement améliore encore l'élimination des molécules responsables de la réponse inflammatoire. L'effet du traitement est beaucoup moins marqué pour les échantillons E5248, E5250, Lab3943 et le NUTRIOSE®. Ces données indiquent que le traitement au charbon SX+ serait efficace pour éliminer les PGN peu dégradés, réduisant ainsi l'activité inflammatoire des matrices pour lesquelles ces contaminants sont majoritaires.

Pour les cellules HEK-TLR4, la réponse est bien diminuée pour les matrices E1565, Lab3943 et NUTRIOSE®, qui sont les plus contaminées en LPS. Toutefois, l'effet n'est pas aussi marqué que pour les réponses attribuées aux PGN, montrant une cinétique spécifique au LPS.

Les traitements au charbon SX+ ont peu d'effet sur la réponse des cellules HEK-NOD2, ce qui indique que les produits de dégradation du PGN ne sont pas correctement éliminés. Finalement, les cellules HEK-Null ne sont pas réactives aux échantillons traités, excluant tout effet toxique lié au charbon.

Les différences observées après traitement par passage sur charbon actif étaient attendues, puisque les tests précédents montrent que les échantillons contiennent des contaminants de nature moléculaire différente. Une information importante apportée par cette expérience est la mise en évidence de différence d'efficacité en fonction de la taille des contaminants. Ainsi, le traitement avec le charbon SX+ aurait un effet plus marquée sur l'élimination d'une certaine catégorie de contaminants, notamment les PGN de haut poids moléculaire.

Afin de confirmer cette hypothèse, plusieurs charbons de porosité différente ont été testés pour leur efficacité à décontaminer les matrices E3063, E1565, E1242 et Lab3943.

Contrairement aux matrices E3063 et E1242 qui sont majoritairement contaminées par des PGN de taille élevée (forte réponse TLR2), les matrices E1565 et Lab3943 contiennent des PGN partiellement dégradés (réponses TLR2 et NOD2) et des LPS (réponse TLR4).

Les conditions de traitement optimisées sont les suivantes : charbon à 0,5%, pH ajusté à 4,5, incubation pendant 1 h à 80°C. Après traitement, les échantillons sont filtrés sur filtre 0,22 µm puis utilisés dans les tests cellulaires.

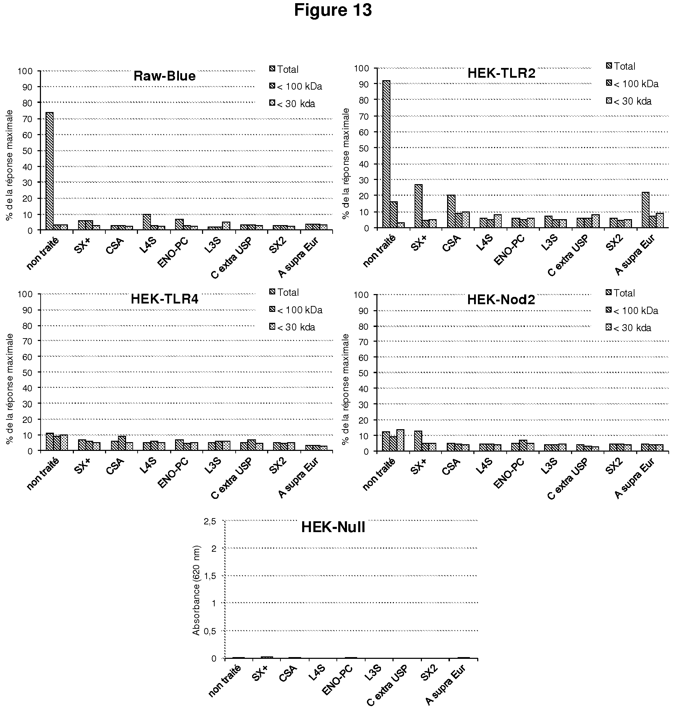

Les résultats pour la matrice E3063 sont présentés dans la figure 13.

L'absence de réponse dans les cellules HEK-Null confirme qu'aucun des charbons ne présentent de toxicité cellulaire.

Tous les charbons testés sont efficaces pour réduire drastiquement les réponses des cellules Raws et HEK-TLR2, ce qui témoigne de leur efficacité pour éliminer les gros PGN, principaux contaminants de cette matrice.

On note que les nouveaux charbons sont équivalents ou plus efficaces que le SX+.

Ainsi, les échantillons traités par L4S, L3S, ENO-PC, C-extra USP et SX2 induisent des réponses cellulaires proches du bruit de fond dans les cellules HEK-TLR2, ce qui suggère que ces charbons sont plus efficaces pour éliminer les PGN.

Concernant les charbons moins efficaces, la filtration sur 30 kDa et 100 kDa réduit les réponses résiduelles après traitement, preuve qu'elles étaient dues essentiellement à des traces de PGN non éliminés.

Les mêmes résultats sont observés dans les cellules Raws, à l'exception de L4S, qui apparait moins efficace, et de A-Supra-Eur, qui au contraire se montre plus efficace que dans les cellules HEK-TLR2. Ce dernier charbon pourrait donc avoir un spectre d'action plus large et éliminer les autres molécules, telles que les LPS.

Même si les charbons diminuent de façon significative la réactivité des cellules HEK-NOD2 et HEK-TLR4 vis-à-vis de la matrice E3063, il est difficile de distinguer des différences d'efficacité entre les traitements en raison de la faible amplitude des réponses induites par cette matrice dans les deux types cellulaires.

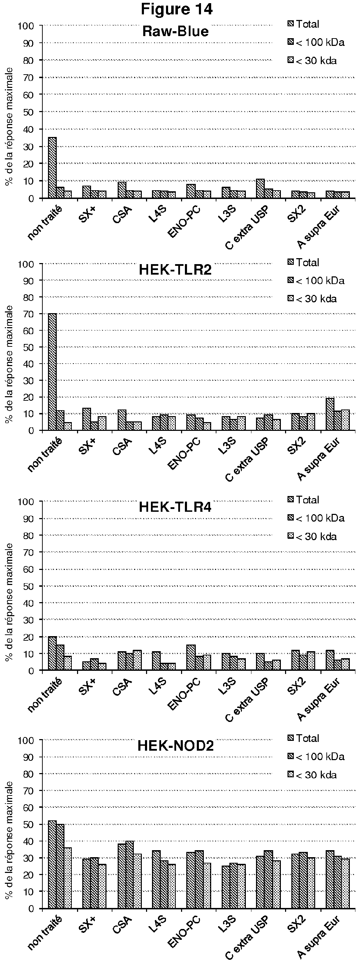

Les résultats pour la matrice E1565 sont présentés dans la figure 14.

Comme pour la matrice E3063, les mêmes charbons sont efficaces pour réduire les réponses des cellules HEK-TLR2 et Raws induites par E1565. Toutefois, on peut noter quelques différences mineures, probablement dues à des différences de tailles et donc de propriétés des PGN.

La quasi-totalité de la réponse des cellules HEK-NOD2 est bien due à la présence des produits de dégradation des PGN, puisque la filtration à 30 kDa ne retient pas ou très peu les contaminants. Par contre, les charbons sont peu efficaces pour réduire la réponse de ces cellules. En effet, la diminution de la réponse provoquée par la réduction de la charge en contaminants de type MDP ne dépasse pas 50% la réponse maximale des cellules.

Finalement, les charbons ont un effet moyen sur la réponse TLR4. A l'exception du SX+ qui est très efficace pour éliminer tous les formes de LPS, la réduction provoquée par le traitement avec les autres charbons n'excède pas 50 % de la réponse induite par le même échantillon non traité. On peut toutefois noter que les charbons L4S, A-supra Eur, et dans une moindre mesure, C-extra USP et ENO-PC, sont plus efficaces pour réduire les réponses induites par les molécules de taille < 100 kDa, ce qui suggère que ces charbons agissent préférentiellement sur les LPS non agrégés.

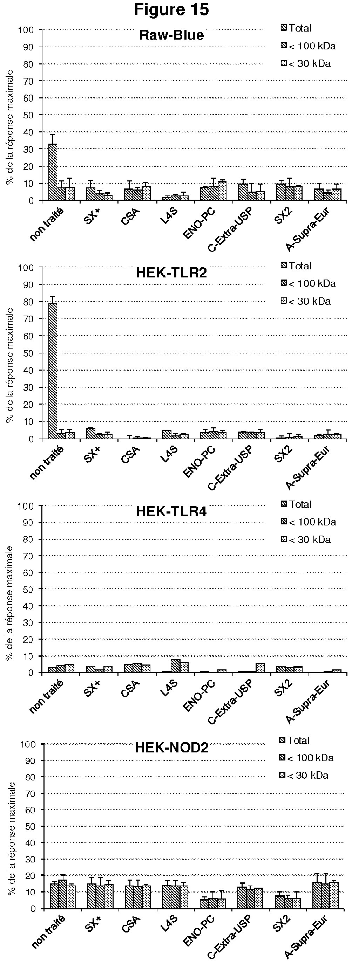

Les résultats pour la matrice E1242 sont présentés dans la figure 15.

Tous les charbons testés sont efficaces pour réduire les réponses induites par la matrice E1242 dans les cellules Raws et HEK-TLR2. Les réponses obtenues, qui sont proches ou égales au bruit de fond, sont identiques avant et après filtration sur 30 kDa et 100 kDa, preuve que les grosses molécules correspondant au PGN ont été éliminées.

La matrice E1242 est très faiblement contaminée par le LPS. Cependant, on peut noter que ENO-PC et A-Supra-Eur sont plus efficaces que les autres charbons pour diminuer la réponse des cellules HEK-TLR4 au niveau du bruit de fond. Cette observation confirme que ces deux charbons ont un spectre d'action large et sont efficaces pour éliminer d'autres molécules que les PGN, telles que les LPS.

Finalement, les charbons ne sont pas efficaces pour éliminer les produits de dégradation des PGN, à l'exception de ENO-PC et SX2, qui réduisent d'environ 50 % la réponse des cellules HEK-NOD2.

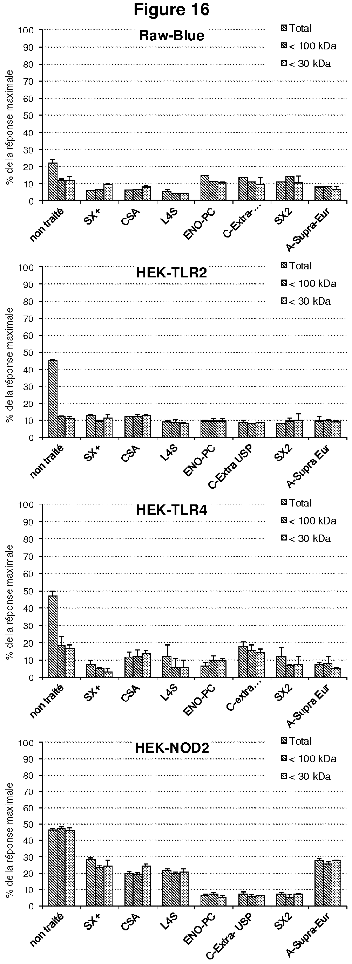

Les résultats pour la matrice Lab3943 sont présentés dans la figure 16.

Cette matrice est contaminée par un large spectre de molécules différentes. Comme attendu, tous les charbons réduisent les réponses induites dans les cellules Raws, mais avec des efficacités différentes. On peut noter que SX+, CSA, L4S et, dans une moindre mesure, A-Supra-Eur, sont les plus efficaces, ce qui confirme que ces charbons ont un spectre d'action large. Pour les cellules HEK-TLR2, les charbons se révèlent tous efficaces pour éliminer les PGN, mais les réponses résiduelles restent identiques avant et après filtration, indiquant que les produits de dégradation des PGN sont plus difficilement éliminés.

Le comportement des charbons dans l'élimination des LPS est également variable. Ainsi, les charbons SX+, ENO-PC, L4S et A-Supra-Eur sont encore les plus efficaces pour réduire la réponse des cellules HEK-TLR4. Finalement, seuls les charbons ENO-PC, C-Extra-USP et SX2 se révèlent relativement actifs pour réduire fortement la réponse des cellules HEK-NOD2, preuve qu'ils sont efficaces pour éliminer les produits de dégradation des PGN présent dans cette matrice.
- C-extra-USP et SX2 : efficaces pour éliminer les PGN et leurs produits de dégradation.
- A-Supra-Eur : spectre large avec efficacité plus élevée pour des molécules de poids moléculaire élevé (ex : LPS agrégés et PGN).
- ENO-PC : spectre large avec efficacité plus élevée pour des molécules de poids moléculaire < 100 kDa (ex : LPS et produits de dégradation des PGN).
- autres charbons : spectres d'action et efficacité au plus équivalentes à celles du charbon SX+.

### Exemple 5 : Effet d'un traitement par ultrafiltration sur 5 kDa.

Le traitement par ultrafiltration a pour objectif de réduire, voire d'éliminer, la contamination par les molécules de petite taille, de façon à contrer leur participation dans le déclenchement d'une réponse inflammatoire, que ce soit par effet direct ou par un phénomène de synergie avec d'autres molécules contaminantes.

Les expériences ont été réalisées sur les matrices E1565 et Lab3943, qui sont toutes deux contaminées par des PGN partiellement dégradés (réponses TLR2 et NOD2) et des LPS (réponse TLR4).

La filtration sur 5 kDa a été réalisée à un débit moyen de 25 mL/min. Les débits des filtrats sont respectivement de 55 mL/h pour E1565 et 65 ml/h pour Lab3943.

Pour vérifier l'efficacité de l'ultrafiltration, les réponses cellulaires ont d'abord été mesurées à partir d'échantillons provenant des fractions Rétentat et Filtrat récupérées après passage de la solution de départ (100 mL).

L'ultrafltration a ensuite été réalisée en circuit fermé avec injection en continu du rétentat dans l'échantillon de départ. Pour compenser la perte de liquide due à l'élimination du filtrat, le volume de l'échantillon a été ajusté en continu au volume de départ par addition d'eau PPI. Dans ce cas, les tests cellulaires ont été réalisés à partir de prélèvements dans l'échantillon effectués après 1 h, 2 h et 3 h d'ultrafiltration.

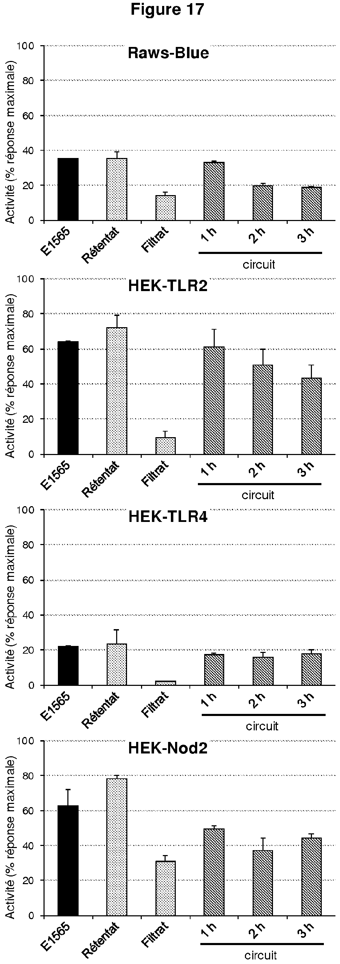

Les résultats pour la matrice E1565 sont présentés dans la figure 17.

Les réponses induites par les fractions Rétentats restent similaires à celles observées pour les échantillons non filtrés dans les quatre tests cellulaires. Toutefois, une réponse inflammatoire significative est observée en réponse aux fractions Filtrats dans les cellules Raws et HEK-NOD2. Ces données sont compatibles avec le seuil de coupure du filtre (5 kDa), qui laisse passer les produits de dépolymérisation du PGN mais pas les PGN et le LPS, surtout si ces derniers sont sous forme d'agrégats. Par contre, l'absence de diminution de réponse inflammatoire dans les Rétentats indique qu'un seul passage sur le filtre est inefficace pour réduire la réactivité inflammatoire de la matrice. En effet, le partage entre les fractions Rétentat/Filtrat est de 25 pour 1, ce qui est insuffisant pour éliminer les petites molécules inflammatoires.

L'ultrafiltration en continu est efficace pour diminuer la réponse induite dans les cellules HEK-NOD2, ce qui était prévisible, mais également dans les cellules Raws et HEK-TLR2. Etant donné la contamination de E1565 par des PGN et du LPS, la diminution de réponse dans les deux derniers types cellulaires est certainement liée à une réduction de l'activité synergique des petites molécules inflammatoires.

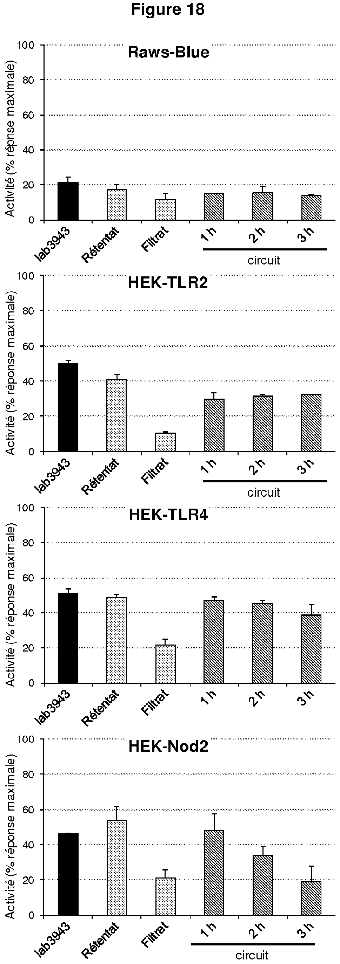

Les résultats pour la matrice Lab3943 sont présentés dans la figure 18.

Comme attendu, une activité inflammatoire associée au MDP est retrouvée dans le Filtrat (HEK-NOD2). Une réponse significative est également observée dans les cellules HEK-TLR4. Ce résultat montre que le LPS est dans une configuration structurale moins agrégée que celui présent dans la matrice E1565, ce qui a permis son passage à travers le filtre.

Comme pour E1565, la filtration en continu est efficace pour diminuer la charge en produits de dépolymérisation des PGN, ce qui est visible par une nette réduction de la réponse des cellules HEK-NOD2 (effet direct) et par une diminution plus faible mais significative de la réactivité des cellules Raws et HEK-TLR2 (action synergique).

### Exemple 6 : Effet des traitements enzymatiques.

L'objectif de ces essais est de tester la capacité d'enzymes industrielles à diminuer la réactivité pro-inflammatoire des contaminants présents dans les matrices de polymère de glucose.

Les échantillons sont préparés à 32 % (poids/volume) et traités en présence des enzymes selon les conditions décrites ci-après. Après traitement, les enzymes sont désactivées par chauffage, les solutions sont filtrées sur filtre stérile 0,22 µm, puis utilisées dans les tests cellulaires.

Trois préparations enzymatiques industrielles ont été testées pour leur capacité à réduire la charge en contaminants :
- Mannaway® : incubation à 0,4 % (vol/vol), pH 10, 50°C, pendant 4 h et 24 h.
- SEBflo® TL : incubation à 0,35 mg/g de matrice, 50°C, pH 5, de 30 min à 24 h.
- SEBPro®FL100 : incubation à 4 % (vol/vol), pH 3, 55°C, de 1 h à 24 h.

Les deux matrices choisies pour les tests sont : E3063 (forte contamination en PGN faiblement dégradés et traces de LPS) et E5250 (faible contamination en PGN et présence de molécules inflammatoires autres que PGN et LPS).

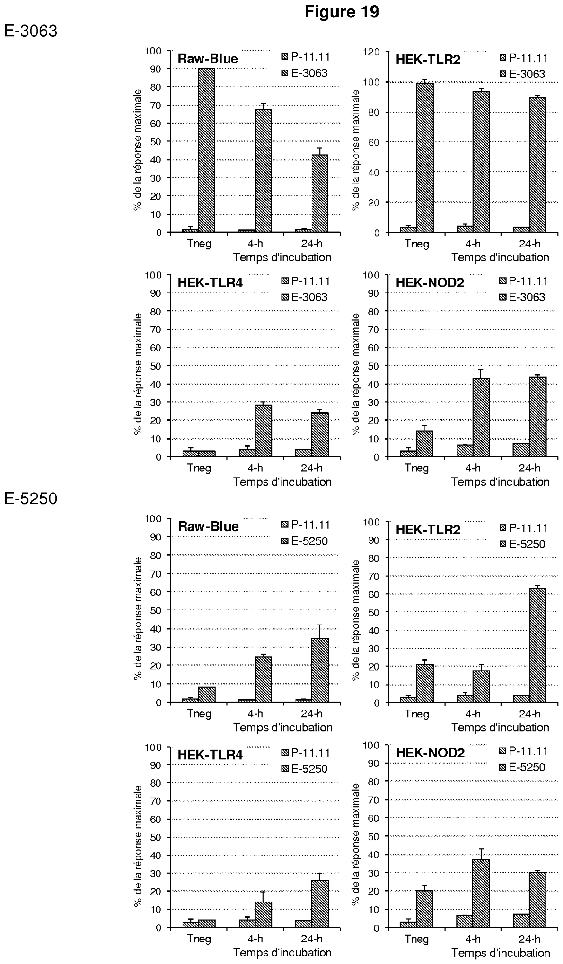

Les effets des traitements des deux matrices par la Mannaway® sont présentés dans la figure 19.

L'addition de la solution enzymatique dans la matrice P-11.11 (témoin de non contamination) n'induit aucune réponse inflammatoire dans les cellules Raws, HEK-TLR2 ou HEK-TLR4. Une faible augmentation est observée dans les cellules HEK-NOD2, suggérant la présence de produits de dégradation des PGN à l'état de traces. Toutefois, ces résultats montrent que l'enzyme utilisée dans les conditions de l'expérience n'apporte pas de contaminants pro-inflammatoires majeurs.

Les essais réalisés sur la matrice E3063 montrent que l'enzyme a probablement une faible action lytique sur les PGN, puisqu'on observe une diminution des réponses dans les cellules Raws et HEK-TLR2. Le traitement provoque une augmentation de la réponse des cellules HEK-NOD2, ce qui est compatible avec une dégradation partielle des PGN. Par contre, une augmentation de la réponse des cellules HEK-TLR4 est également observée. Etant donné que la préparation enzymatique n'apporte pas de contamination, l'apparition de LPS est probablement due à un relargage de ce contaminant à partir de la matrice elle-même.

Le traitement enzymatique de la matrice E5250 induit une augmentation de la réponse inflammatoire dans les quatre types cellulaire. A l'origine, cette matrice déclenchait de faibles réponses inflammatoires. Par conséquent, l'augmentation des réponses TLR2 et TRL4 suggère que l'enzyme a libéré des contaminants de type PGN et LPS à partir de la matrice.

La Mannaway® est couramment utilisée comme agent de clarification des produits agro-alimentaires. Les résultats obtenus suggèrent que l'enzyme a probablement dissocié des macro-complexes (débris bactériens) qui étaient normalement éliminés par l'étape de filtration sur filtre 0,22 µm. En solubilisant ces molécules inflammatoires, l'enzyme les a rendu accessibles pour induire des réponses dans les tests cellulaires.

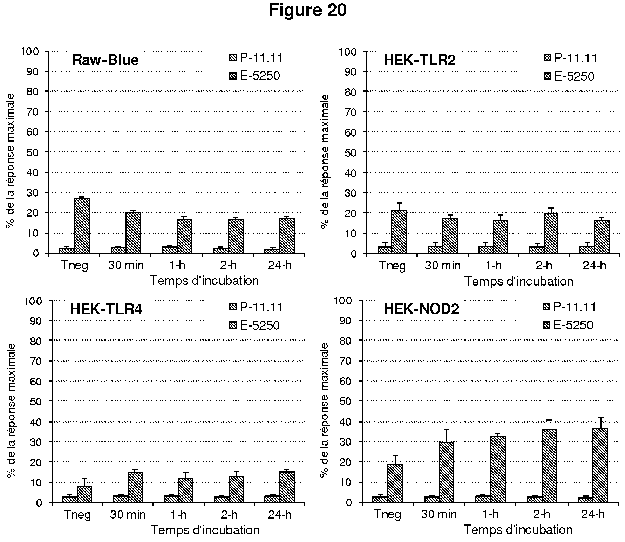

Les effets des traitements de la matrice E5250 par la SEBflo®TL sont présentés dans la figure 20.

L'addition de la solution enzymatique dans la matrice P-11.11 n'induit aucune réponse inflammatoire dans les quatre types cellulaires, preuve que l'enzyme utilisée dans les conditions de l'expérience n'apporte pas de contaminants.

Le traitement de la matrice E5250 induit une faible diminution des réponses inflammatoires dans les cellules Raws et HEK-TLR2. L'enzyme est décrit essentiellement pour ses propriétés beta-glucanase. Toutefois, la diminution des réponses cellulaires observées s'accompagne d'une augmentation de la réponse des cellules HEK-NOD2. Ces données indiquent que la préparation enzymatique contient également une activité capable de dégrader les PGN.

Parallèlement à l'apparition des produits de dégradation des PGN, une faible augmentation de la réponse TLR4 est observée en présence de l'enzyme, alors que cette dernière est non contaminée. Comme précédemment, l'enzyme a probablement libéré des contaminants inflammatoires, mais à un degré moindre que ce qui est observé avec la Mannaway®.

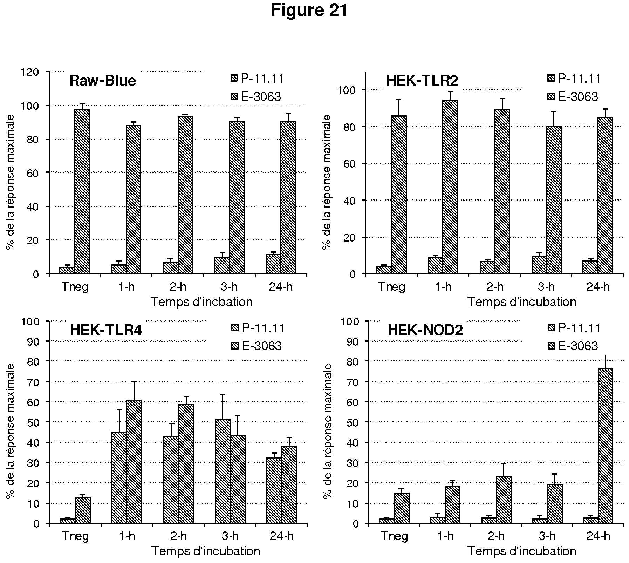

Les effets des traitements de la matrice E3063 par la SEBPro® FL100 sont présentés dans la figure 21.

L'addition de la préparation enzymatique dans la matrice P-11.11 induit une forte réponse inflammatoire dans les cellules HEK-TLR4, ainsi que des réponses faibles mais significative dans les cellules Raws et HEK-TLR2. Ces données indiquent que l'enzyme est contaminée par du LPS et des traces de PGN.

L'addition de l'enzyme à la matrice E3063 induit une faible diminution de la réponse TLR2, qui s'accompagne d'une augmentation de la réponse des cellules HEK-NOD2. Ces données indiquent que la SEBPro®FL100 a une faible action lytique sur les PGN. Toutefois, son utilisation nécessiterait obligatoirement une étape préalable de décontamination pour éliminer le LPS.

### Exemple 7 : Effet des traitements par passage sur résines.

L'objectif de ces essais est de tester la capacité de résines industrielles à retenir les contaminants présents dans les matrices de polymère de glucose et par conséquent à réduire la réactivité pro-inflammatoire de ces matrices.

Les essais ont été réalisés avec la matrice E1565 (solubilisée à 32% poids/volume dans l'eau stérile), car elle est contaminée par les différents types de molécules pro-inflammatoires susceptibles d'être retrouvées dans les circuits de production (réponses TLR2, TLR4 ET NOD2).

Pour les expériences, la solution à décontaminer a été éluée en continu sur une colonne contenant 20 mL de chaque résine (*bed volume*). Les tests cellulaires de réactivité inflammatoire ont été réalisés à partir de la solution avant passage sur la colonne (témoin de contamination), puis sur les échantillons récupérés après passage de 4 volumes (passage 5) et de 10 volumes (passage 11) de solution. Cette procédure a permis de vérifier si la présence du polymère de glucose ne provoquait pas un phénomène de la saturation des résines.

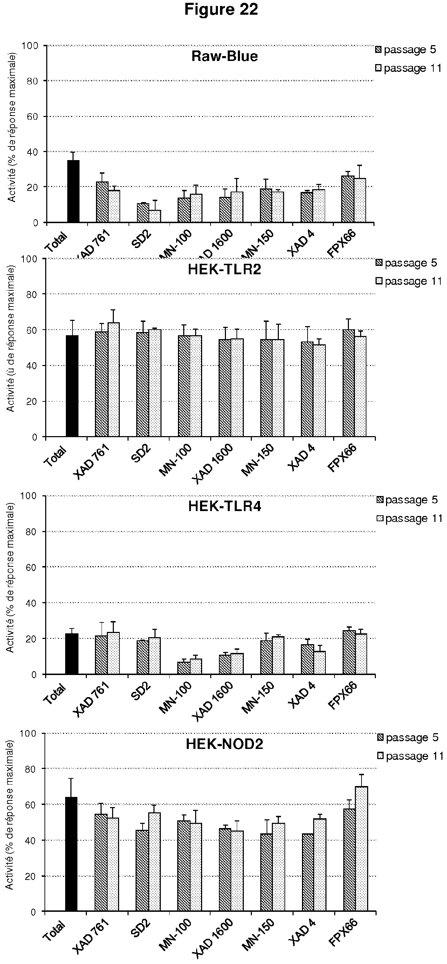

Les résultats sont présentés dans la figure 22.

Le passage de la solution sur les différentes résines provoque une diminution de la réactivité inflammatoire de la matrice E1565 au contact des cellules Raws. A l'exception de FPX66, la diminution de réponse atteint au moins 50% pour les autres résines. La réduction atteint même 70% après passage sur la résine SD2, ce qui indique que cette résine est la plus efficace pour éliminer les molécules contaminantes à activité pro-inflammatoire contenues dans la matrice E1565. Dans tous les cas, aucune différence significative n'est observée entre les passages 5 et 11, ce qui exclut un quelconque phénomène de saturation du support.

La réactivité des cellules HEK-TLR2 vis-à-vis de la matrice E5250 n'est pas significativement modifiée après passage sur les différentes résines, ce qui indique que ces traitements sont inefficaces pour réduire la charge en PGN contaminant.

Les résines MN-100 et XAD-1600 se montrent quant à elles très efficaces pour réduire les réponses des HEK-TLR4 vis-à-vis de la matrice E1565. Ces données indiquent que ces deux résines possèdent une capacité élevée de rétention des molécules de type LPS. A l'inverse, les autres résines sont peu, voire totalement inefficaces, pour retenir ce contaminant.

Finalement, les différentes résines réduisent modérément les réponses des cellules HEK-NOD2 vis-à-vis de la matrice, à l'exception de FPX66 qui est totalement inefficace. L'effet observé reste toutefois faible, démontrant que les résines ont une faible capacité de rétention des produits de dégradation des PGN.

Hormis la présence de traces de LPS, la matrice E1565 est fortement contaminée par du PGN et par ses produits de dégradation. Ces derniers ont peu de réactivité inflammatoire *per se,* par contre, ils sont capables d'agit en synergie avec les autres molécules inflammatoires interagissant avec les TLRs, tels que les PGN et les LPS, et d'exacerber la réponse inflammatoire globale.

Les tests réalisés dans cet exemple montrent une diminution significative de la réactivité des cellules Raws après passage sur les différentes résines. Cette diminution de réponse inflammatoire globale n'est pas consécutive à une rétention des PGN, puisque le passage sur résines ne modifie pas les réponses TLR2.

Seules deux résines (MN-100, XAD-1600) sur sept sont nettement efficaces pour réduire la réponse TLR4. On peut donc en déduire que la diminution de réponse inflammatoire observée dans les cellules Raws est consécutive, du moins en partie, à la rétention du LPS après passage de la matrice sur ces deux résines.

A l'exception de FPX66, toutes les résines retiennent modérément les produits de dégradation des PGN. Etant donné l'impact de ces petites molécules sur l'exacerbation des réponses inflammatoires, ces résultats indiquent que l'effet majeur des résines est d'éliminer les effets synergiques associés à ces petites molécules.

Quant à la matrice FPX66, son effet est probablement lié à l'élimination de molécules inflammatoires autres que les LPS, les PGN et leurs produits de dégradation. Cette hypothèse est compatible avec le fait que cette résine est la moins efficace pour réduite la réponse inflammatoire globale.

Dans leur ensemble, ces données indiquent qu'un traitement des matrices avec certaines résines industrielles judicieusement sélectionnées peut se révéler efficace pour éliminer les contaminants inflammatoires autres que les PGN, mais également réduire les effets de synergie observés entre ces molécules. L'exemple 4 de la présente étude a montré que certains charbons industriels sont particulièrement efficaces pour éliminer les PGN. Par conséquent, une procédure associant les deux types de traitements devrait permettre de cibler les différentes familles de contaminants et de proposer des matrices exemptes de réactivité inflammatoire.

## Revendications

1. Procédé permettant de tester l'effet d'étape(s) de production ou l'efficacité d'étape(s) de purification sur la présence ou la nature des molécules pro-inflammatoires dans des polymères de glucose ou leurs hydrolysats, comprenant :
a) fournir des polymères de glucose ou leurs hydrolysats ;
b) détecter ou doser les molécules pro-inflammatoires dans les polymères de glucose ou leurs hydrolysats fournis à l'étape a) ;
c) effectuer la ou les étapes de production ou de purification sur les polymères de glucose ou leurs hydrolysats fournis à l'étape a) ;
d) détecter ou doser les molécules pro-inflammatoires dans les polymères de glucose ou leurs hydrolysats obtenus après l'étape c) ;
e) déterminer l'efficacité ou l'impact de l'étape c) sur la présence ou la nature des molécules pro-inflammatoires par comparaison des molécules pro-inflammatoires dans les polymères de glucose ou leurs hydrolysats détectées ou dosées aux étapes b) et d), une diminution de la quantité de molécules pro-inflammatoires ou de certaines de ces molécules étant indicative de l'efficacité de l'étape c) pour la décontamination des polymères de glucose ou de leurs hydrolysats;
dans lequel l'étape de détection ou dosage des molécules pro-inflammatoires dans les polymères de glucose ou leurs hydrolysats comprend un test *in vitro* de réponse inflammatoire à l'aide d'une lignée cellulaire exprimant le récepteur TLR2 (Toll Like Receptor 2) et un gène rapporteur dont la transcription est sous la dépendance directe de la voie de signalisation associée au récepteur TLR2, les molécules pro-inflammatoires étant détectées ou dosées par mesure de l'activité ou du signal du gène rapporteur.

2. Procédé optimisé de production ou de purification de polymères de glucose ou de leurs hydrolysats, comprenant :
a) fournir des polymères de glucose ou leurs hydrolysats ;
b) détecter ou doser les molécules pro-inflammatoires dans les polymères de glucose ou leurs hydrolysats fournis à l'étape a) ;
c) sélectionner la ou les étapes de production ou de purification des polymères de glucose ou de leurs hydrolysats adaptée(s) aux molécules pro-inflammatoires présentes dans les polymères de glucose ou leurs hydrolysats ;
d) effectuer la ou les étapes de production ou de purification sélectionnée(s) sur les polymères de glucose ou leurs hydrolysats fournis à l'étape a) ; et
e) détecter ou doser les molécules pro-inflammatoires dans les polymères de glucose ou leurs hydrolysats obtenus après l'étape d) ;
dans lequel l'étape de détection ou dosage des molécules pro-inflammatoires dans les polymères de glucose ou leurs hydrolysats comprend un test *in vitro* de réponse inflammatoire à l'aide d'une lignée cellulaire exprimant le récepteur TLR2 (Toll Like Receptor 2) et un gène rapporteur dont la transcription est sous la dépendance directe de la voie de signalisation associée au récepteur TLR2, les molécules pro-inflammatoires étant détectées ou dosées par mesure de l'activité ou du signal du gène rapporteur.

3. Procédé selon l'une quelconque des revendications précédentes, dans lequel le test *in vitro* de réponse inflammatoire comprend en outre la mise en contact des polymères de glucose ou de leurs hydrolysats avec la lignée cellulaire THP-1 différenciée en macrophage, sensibilisée par MDP ou LPS, les molécules pro-inflammatoires étant détectées ou dosées par mesure de la quantité de RANTES ou TNF-α produite par la lignée cellulaire.

4. Procédé selon l'une quelconque des revendications précédentes, dans lequel le test *in vitro* de réponse inflammatoire comprend en outre la mise en contact des polymères de glucose ou de leurs hydrolysats avec une lignée de macrophages transfectée avec un gène rapporteur dont la transcription est sous la dépendance directe des voies de signalisation inflammatoires, les molécules pro-inflammatoires étant détectées ou dosées par mesure de l'activité ou du signal du gène rapporteur.

5. Procédé selon l'une quelconque des revendications précédentes, dans lequel le test *in vitro* de réponse inflammatoire comprend en outre la mise en contact des polymères de glucose ou de leurs hydrolysats avec une lignée cellulaire exprimant le récepteur TLR4 et un gène rapporteur dont la transcription est sous la dépendance directe des voies de signalisation de TLR4, les molécules pro-inflammatoires étant détectées ou dosées par mesure de l'activité ou du signal du gène rapporteur.

6. Procédé selon l'une quelconque des revendications précédentes, dans lequel le test *in vitro* de réponse inflammatoire comprend en outre la mise en contact des polymères de glucose ou de leurs hydrolysats avec une lignée cellulaire exprimant le récepteur NOD2 et un gène rapporteur dont la transcription est sous la dépendance directe des voies de signalisation de NOD2, les molécules pro-inflammatoires étant détectées ou dosées par mesure de l'activité ou du signal du gène rapporteur.

7. Procédé selon l'une quelconque des revendications précédentes, dans lequel le test *in vitro* de réponse inflammatoire comprend la mise en contact des polymères de glucose ou de leurs hydrolysats avec :
a. la lignée cellulaire THP-1 différenciée en macrophage, sensibilisée par MDP ou LPS, les molécules pro-inflammatoires étant détectées ou dosées par mesure de la quantité de RANTES ou TNF-α produite par la lignée cellulaire ; et,
b. une lignée de macrophages transfectée avec un gène rapporteur dont la transcription est sous la dépendance directe des voies de signalisation inflammatoires, les molécules pro-inflammatoires étant détectées ou dosées par mesure de l'activité ou du signal du gène rapporteur ; et,
c. une lignée cellulaire exprimant le récepteur TLR2 et un gène rapporteur dont la transcription est sous la dépendance directe des voies de signalisation de TLR2, les molécules pro-inflammatoires étant détectées ou dosées par mesure de l'activité ou du signal du gène rapporteur ; et
d. une lignée cellulaire exprimant le récepteur NOD2 et un gène rapporteur dont la transcription est sous la dépendance directe des voies de signalisation de NOD2, les molécules pro-inflammatoires étant détectées ou dosées par mesure de l'activité ou du signal du gène rapporteur ; et,
e. une lignée cellulaire exprimant le récepteur TLR4 et un gène rapporteur dont la transcription est sous la dépendance directe des voies de signalisation de TLR2, les molécules pro-inflammatoires étant détectées ou dosées par mesure de l'activité ou du signal du gène rapporteur.

8. Procédé selon l'une quelconque des revendications précédentes, dans lequel le test *in vitro* de réponse inflammatoire comprend en outre la mise en contact des polymères de glucose ou de leurs hydrolysats avec une lignée contrôle non transfectée par un récepteur de l'immunité.

9. Procédé selon l'une quelconque des revendications précédentes, dans lequel les molécules pro-inflammatoires sont des molécules d'origine bactérienne, de préférence sélectionnées parmi PGN, des LPS, des lipopeptides, des produits de dépolymérisation des PGN, notamment du MDP, les peptides microbiens formylés comme le f-MLP, et des β glucanes.

10. Procédé selon l'une quelconque des revendications précédentes, dans lequel la ou les étapes de production ou de purification sont choisies parmi des étapes de traitement thermique, d'acidification, de passage sur charbon actif, de passage sur des résines d'adsorption, d'ultrafiltration, de filtration, d'hydrolyse chimique ou enzymatique, ou des combinaisons de celles-ci.

11. Procédé selon l'une quelconque des revendications précédentes, dans lequel les polymères de glucose sont sélectionnés parmi de l'icodextrine et des maltodextrines, en particulier des maltodextrines branchées ou non, et les hydrolysats de polymères de glucose sont un produit d'hydrolyse totale tel le dextrose monohydrate.

12. Procédé selon l'une quelconque des revendications précédentes, dans lequel les échantillons de polymères de glucose ou de leurs hydrolysats sont préalablement filtrés, notamment avec un seuil de coupure à 30 kDa, et le filtrat est mis en contact avec la lignée cellulaire utilisée dans le test.

## Patentansprüche

1. Verfahren zum Testen des Effekts von einem Produktionsschritt oder Produktionsschritten oder der Effizienz von einem Reinigungsschritt oder Reinigungsschritten auf das Vorhandensein oder die Art der pro-inflammatorischen Moleküle in Glucosepolymeren oder ihren Hydrolysaten, umfassend:
a) Bereitstellen der Glucosepolymere oder ihrer Hydrolysate;
b) Nachweis oder quantitative Bestimmung der pro-inflammatorischen Moleküle in den in Schritt a) bereitgestellten Glucosepolymeren oder ihren Hydrolysaten;
c) Durchführen des oder der Produktions- oder Reinigungsschritte mit den in Schritt a) bereitgestellten Glucosepolymeren oder ihren Hydrolysaten;
d) Nachweis oder quantitative Bestimmung der pro-inflammatorischen Moleküle in den nach Schritt c) erhaltenen Glucosepolymeren oder ihren Hydrolysaten;
e) Bestimmen der Effizienz oder der Wirkung von Schritt c) auf das Vorhandensein oder die Art der pro-inflammatorischen Moleküle durch Vergleich der in den Schritten b) und d) nachgewiesenen oder quantitativ bestimmten pro-inflammatorischen Moleküle in den Glucosepolymeren oder ihren Hydrolysaten, wobei eine Verringerung der Menge an pro-inflammatorischen Moleküle oder einiger dieser Moleküle ein Hinweis auf die Effizienz des Schrittes c) für die Dekontaminierung der Glucosepolymere oder ihrer Hydrolysate ist;
wobei der Schritt des Nachweises oder der quantitativen Bestimmung der pro-inflammatorischen Moleküle in den Glucosepolymeren oder ihren Hydrolysaten einen *in vitro* Test der inflammatorischen Reaktion mithilfe einer Zelllinie umfasst, die den TLR2-Rezeptor (Toll Like Receptor 2) und ein Reportergen exprimiert, dessen Transkription von dem TLR2-Rezeptor-assoziierten Signalweg direkt abhängig ist, wobei die pro-inflammatorischen Moleküle durch Messung der Aktivität oder des Signals des Reportergens nachgewiesen oder quantitativ bestimmt werden.

2. Optimiertes Verfahren zur Produktion oder Reinigung von Glucosepolymeren oder ihren Hydrolysaten, umfassend:
a) Bereitstellen der Glucosepolymere oder ihrer Hydrolysate;
b) Nachweis oder quantitative Bestimmung der pro-inflammatorischen Moleküle in den in Schritt a) bereitgestellten Glucosepolymeren oder ihren Hydrolysaten;
c) Auswählen des oder der Produktions- oder Reinigungsschritte der Glucosepolymere oder ihrer Hydrolysate, die für die pro-inflammatorischen Moleküle geeignet sind, die in den Glucospolymeren und ihren Hydrolysaten vorhanden sind;
d) Durchführen des oder der Produktions- oder Reinigungsschritte, die für die in Schritt a) bereitgestellten Glucosepolymere und ihre Hydrolysate ausgewählt sind; und
e) Nachweis oder quantitative Bestimmung der pro-inflammatorischen Moleküle in den nach Schritt c) erhaltenen Glucosepolymeren oder ihren Hydrolysaten;
wobei der Schritt des Nachweises oder der quantitativen Bestimmung der pro-inflammatorischen Moleküle in den Glucosepolymeren oder ihren Hydrolysaten einen *in vitro* Test der inflammatorischen Reaktion mithilfe einer Zelllinie umfasst, die den TLR2-Rezeptor (Toll Like Receptor 2) und ein Reportergen exprimiert, dessen Transkription von dem TLR2-Rezeptor-assoziierten Signalweg direkt abhängig ist, wobei die pro-inflammatorischen Moleküle durch Messung der Aktivität oder des Signals des Reportergens nachgewiesen oder quantitativ bestimmt werden.

3. Verfahren gemäß einem der vorhergehenden Ansprüche, wobei der *in vitro* Test der inflammatorischen Reaktion außerdem das Inkontaktbringen der Glucosepolymere oder ihrer Hydrolysate mit der THP-1-Zelllinie umfasst, die zu MDP- oder LPS-sensibilisierten Makrophagen ausdifferenziert ist, wobei die pro-inflammatorischen Moleküle durch Messung der von der Zelllinie produzierten Menge an RANTES oder TNF-α, nachgewiesen oder quantitativ bestimmt werden.

4. Verfahren gemäß einem der vorhergehenden Ansprüche, wobei der *in vitro* Test der inflammatorischen Reaktion außerdem das Inkontaktbringen der Glucosepolymere oder ihrer Hydrolysate mit einer Makrophagenlinie umfasst, die mit einem Reportergen transfiziert ist, dessen Transkription von den inflammatorischen Signalwegen direkt abhängig ist, wobei die pro-inflammatorischen Moleküle durch Messung der Aktivität oder des Signals des Reportergens nachgewiesen oder quantitativ bestimmt werden.

5. Verfahren gemäß einem der vorhergehenden Ansprüche, wobei der *in vitro* Test der inflammatorischen Reaktion außerdem das Inkontaktbringen der Glucosepolymere oder ihrer Hydrolysate mit einer Zelllinie umfasst, die den TLR4-Rezeptor und ein Reportergen exprimiert, dessen Transkription von den TLR4-Signalwegen direkt abhängig ist, wobei die pro-inflammatorischen Moleküle durch Messung der Aktivität oder des Signals des Reportergens nachgewiesen oder quantitativ bestimmt werden.

6. Verfahren gemäß einem der vorhergehenden Ansprüche, wobei der *in vitro* Test der inflammatorischen Reaktion außerdem das Inkontaktbringen der Glucosepolymere oder ihrer Hydrolysate mit einer Zelllinie umfasst, die den NOD2-Rezeptor und ein Reportergen exprimiert, dessen Transkription von den NOD2-Signalwegen direkt abhängig ist, wobei die pro-inflammatorischen Moleküle durch Messung der Aktivität oder des Signals des Reportergens nachgewiesen oder quantitativ bestimmt werden.

7. Verfahren gemäß einem der vorhergehenden Ansprüche, wobei der *in vitro* Test der inflammatorischen Reaktion das Inkontaktbringen der Glucosepolymere oder ihrer Hydrolysate umfasst mit:
a) der THP-1-Zelllinie, die zu MDP- oder LPS-sensibilisierten Makrophagen ausdifferenziert ist, wobei die pro-inflammatorischen Moleküle durch Messung der von der Zelllinie produzierten Menge an RANTES oder TNF-α, nachgewiesen oder quantitativ bestimmt werden; und
b) einer Makrophagenlinie, die mit einem Reportergen transfiziert ist, dessen Transkription von den inflammatorischen Signalwegen direkt abhängig ist, wobei die pro-inflammatorischen Moleküle durch Messung der Aktivität oder des Signals des Reportergens nachgewiesen oder quantitativ bestimmt werden; und
c) einer Zelllinie, die den TLR2-Rezeptor und ein Reportergen exprimiert, dessen Transkription von den TLR2-Signalwegen direkt abhängig ist, wobei die pro-inflammatorischen Moleküle durch Messung der Aktivität oder des Signals des Reportergens nachgewiesen oder quantitativ bestimmt werden; und
d) einer Zelllinie, die den NOD2-Rezeptor und ein Reportergen exprimiert, dessen Transkription von den NOD2-Signalwegen direkt abhängig ist, wobei die pro-inflammatorischen Moleküle durch Messung der Aktivität oder des Signals des Reportergens nachgewiesen oder quantitativ bestimmt werden; und
e) einer Zelllinie, die den TLR4-Rezeptor und ein Reportergen exprimiert, dessen Transkription von den TLR4-Signalwegen direkt abhängig ist, wobei die pro-inflammatorischen Moleküle durch Messung der Aktivität oder des Signals des Reportergens nachgewiesen oder quantitativ bestimmt werden.

8. Verfahren gemäß einem der vorhergehenden Ansprüche, wobei der *in vitro* Test der inflammatorischen Reaktion außerdem das Inkontaktbringen der Glucosepolymere oder ihrer Hydrolysate mit einer Kontrollzelllinie umfasst, die nicht mit einem Immunrezeptor transfiziert ist.

9. Verfahren gemäß einem der vorhergehenden Ansprüche, wobei die pro-inflammatorischen Moleküle bakteriellen Ursprungs sind, vorzugsweise ausgewählt aus PGN, LPS, Lipopeptiden, Depolymerisationsprodukten von PGN, insbesondere MDP, formylierten mikrobiellen Peptiden, wie f-MLP, und β-Glucanen.

10. Verfahren gemäß einem der vorhergehenden Ansprüche, wobei der oder die Produktions- oder Reinigungsschritte aus Schritten der thermischen Behandlung, Ansäuerung, Passage durch Aktivkohle, Passage über Adsorptionsharze, Ultrafiltration, Filtration, chemischen oder enzymatischen Hydrolyse oder Kombinationen davon ausgewählt sind.

11. Verfahren gemäß einem der vorhergehenden Ansprüche, wobei die Glucosepolymere aus Icodextrin und Maltodextrinen, insbesondere verzweigten oder nichtverzweigten Maltodextrinen, ausgewählt sind und die Hydrolysate der Glucosepolymere ein Produkt der vollständigen Hydrolyse wie Dextrosemonohydrat sind.

12. Verfahren gemäß einem der vorhergehenden Ansprüche, wobei die Proben der Glucosepolymere oder ihrer Hydrolysate zuvor filtriert werden, insbesondere mit einer Ausschlussgrenze von 30 kDa, und das Filtrat mit der im Test eingesetzten Zelllinie in Kontakt gebracht wird.

## Claims

1. A method for testing the effect of a production step or production steps or the effectiveness of a purification step or purification steps on the presence or the nature of pro-inflammatory molecules in glucose polymers or hydrolysates thereof, comprising:
a) providing glucose polymers or hydrolysates thereof;
b) detecting or assaying the pro-inflammatory molecules in the glucose polymers or hydrolysates thereof provided in step a);
c) carrying out the production or purification step or steps on the glucose polymers or hydrolysates thereof provided in step a);
d) detecting or assaying the pro-inflammatory molecules in the glucose polymers or hydrolysates thereof obtained after step c);
e) determining the effectiveness or the impact of step c) on the presence or the nature of the pro-inflammatory molecules for glucose polymers or hydrolysates thereof detected or assayed at steps b) and d), a decrease of the amount of pro-inflammatory molecules or some of these pro-inflammatory molecules being indicative of the efficie,cy of step c) for decontaminating glucose polymers or hydrolysates thereof;
in which the step for detecting or assaying the pro-inflammatory molecules in the glucose polymers or hydrolysates thereof comprises an *in vitro* inflammatory response test using a cell line expressing the TLR2 receptor (Toll Like Receptor 2) and a reporter gene the transcription of which is under the direct control of the TLR2 signaling pathway, the pro-inflammatory molecules being detected or assayed by measuring the activity or the signal of the reporter gene.

2. Optimized method for producing or purifying glucose polymers or hydrolysates thereof, comprising:
a) providing glucose polymers or hydrolysates thereof;
b) detecting or assaying the pro-inflammatory molecules in the glucose polymers or hydrolysates thereof provided in step a);
c) selecting the step or steps for producing or purifying the glucose polymers or hydrolysates thereof that is or are suitable for the pro-inflammatory molecules present in the glucose polymers or hydrolysates thereof;
d) carrying out the selected production or purification step or steps on the glucose polymers or hydrolysates thereof provided in step a); and
e) detecting or assaying the pro-inflammatory molecules in the glucose polymers or hydrolysates thereof obtained after step d);
in which the step for detecting or assaying the pro-inflammatory molecules in the glucose polymers or hydrolysates thereof comprises an *in vitro* inflammatory response test using a cell line expressing the TLR2 receptor (Toll Like Receptor 2) and a reporter gene the transcription of which is under the direct control of the TLR2 signaling pathway, the pro-inflammatory molecules being detected or assayed by measuring the activity or the signal of the reporter gene.

3. The method as claimed in any one of the preceding claims, wherein the *in vitro* inflammatory response test further comprises bringing the glucose polymers or hydrolysates thereof into contact with the MDP- or LPS-sensitized, macrophage-differentiated THP-1 cell line, the pro-inflammatory molecules being detected or assayed by measuring the amount of RANTES or TNF-α produced by the cell line.

4. The method as claimed in any one of the preceding claims, wherein the *in vitro* inflammatory response test further comprises bringing the glucose polymers or hydrolysates thereof into contact with a macrophage line transfected with a reporter gene, the transcription of which is under the direct control of the inflammatory signaling pathways, the pro-inflammatory molecules being detected or assayed by measuring the activity or the signal of the reporter gene.

5. The method as claimed in any one of the preceding claims, wherein the *in vitro* inflammatory response test further comprises bringing the glucose polymers or hydrolysates thereof into contact with a cell line expressing the TLR4 receptor and a reporter gene, the transcription of which is under the direct control of the TLR4 signaling pathways, the pro-inflammatory molecules being detected or assayed by measuring the activity or the signal of the reporter gene.

6. The method as claimed in any one of the preceding claims, wherein the *in vitro* inflammatory response test further comprises bringing the glucose polymers or hydrolysates thereof into contact with a cell line expressing the NOD2 receptor and a reporter gene, the transcription of which is under the direct control of the NOD2 signaling pathways, the pro-inflammatory molecules being detected or assayed by measuring the activity or the signal of the reporter gene.

7. The method as claimed in any one of the preceding claims, wherein the *in vitro* inflammatory response test comprises bringing the glucose polymers or hydrolysates thereof into contact with:
a. the MDP- or LPS-sensitized, macrophage-differentiated THP-1 cell line, the pro-inflammatory molecules being detected or assayed by measuring the amount of RANTES or TNF-α produced by the cell line; and
b. a macrophage line transfected with a reporter gene, the transcription of which is under the direct control of the inflammatory signaling pathways, the pro-inflammatory molecules being detected or assayed by measuring the activity or the signal of the reporter gene; and
c. a cell line expressing the TLR2 receptor and a reporter gene, the transcription of which is under the direct control of the TLR2 signaling pathways, the pro-inflammatory molecules being detected or assayed by measuring the activity or the signal of the reporter gene; and
d. a cell line expressing the NOD2 receptor and a reporter gene, the transcription of which is under the direct control of the NOD2 signaling pathways, the pro-inflammatory molecules being detected or assayed by measuring the activity or the signal of the reporter gene; and
e. a cell line expressing the TLR4 receptor and a reporter gene, the transcription of which is under the direct control of the TLR4 signaling pathways, the pro-inflammatory molecules being detected or assayed by measuring the activity or the signal of the reporter gene.

8. The method as claimed in any one of the preceding claims, wherein the *in vitro* inflammatory response test further comprises bringing the glucose polymers or hydrolysates thereof into contact with a control line not transfected with an immunity receptor.

9. The method as claimed in any one of the preceding claims, wherein the pro-inflammatory molecules are molecules of bacterial origin, preferably selected from PGNs, LPSs, lipopeptides, PGN depolymerization products, in particular MDP, formylated microbial peptides such as f-MLP, and β-glucans.

10. The method as claimed in any one of the preceding claims, wherein the production or purification step or steps is or are chosen from steps of heat treatment, of acidification, of passing over activated carbon, of passing over adsorption resins, of ultrafiltration, of filtration, or of chemical or enzymatic hydrolysis, or combinations thereof.

11. The method as claimed in any one of the preceding claims, wherein the glucose polymers are selected from icodextrin and maltodextrins, in particular branched or unbranched maltodextrins, and the glucose polymer hydrolysates are a product of total hydrolysis, such as dextrose monohydrate.

12. The method as claimed in any one of the preceding claims, wherein the samples of glucose polymers or of hydrolysates thereof are prefiltered, in particular with a cut-off threshold at 30 kDa, and the filtrate is brought into contact with the cell line used in the test.
